(19) Europäisches Patentamt
European Patent Office
Office européen des brevets

(11) EP 2 325 178 A1

(12) **EUROPEAN PATENT APPLICATION**
published in accordance with Art. 153(4) EPC

(43) Date of publication:
**25.05.2011 Bulletin 2011/21**

(21) Application number: **09802755.0**

(22) Date of filing: **23.03.2009**

(51) Int Cl.:
***C07D 317/16*** (2006.01) ***C07D 317/18*** (2006.01)
***C07D 409/04*** (2006.01)

(86) International application number:
**PCT/JP2009/055714**

(87) International publication number:
**WO 2010/013510 (04.02.2010 Gazette 2010/05)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **01.08.2008 JP 2008200225**
**12.09.2008 JP 2008235746**

(71) Applicant: **DAISO CO., LTD.**
**Nishi-ku**
**Osaka-shi**
**Osaka 550-0011 (JP)**

(72) Inventors:
- **ISHII, Yutaka**
**Osaka-shi**
**Osaka 550-0011 (JP)**
- **IWASHINA, Shingo**
**Osaka-shi**
**Osaka 550-0011 (JP)**
- **KAWASE, Takahiro**
**Osaka-shi**
**Osaka 550-0011 (JP)**
- **MATSUI, Yumiko**
**Osaka-shi**
**Osaka 550-0011 (JP)**

(74) Representative: **Duckworth, Timothy John et al**
**J.A. Kemp & Co.**
**14 South Square**
**Gray's Inn**
**London WC1R 5JJ (GB)**

# (54) PROCESS FOR PRODUCTION OF OPTICALLY ACTIVE COMPOUND

(57) 2-Aryl-2-halomethyl-4-chloromethyl-1,3-dioxolane (A) is produced with ease without decreasing the optical purity, by reacting optically active monochlorohydrin and aryl(halomethyl)ketone as starting materials in an acid catalyst. Furthermore, optically active (2-aryl-2-halomethyl-1,3-dioxolan-4-yl)methyl carboxylate and optically active (2-aryl-2-halomethyl-1,3-dioxolan-4-yl)methyl sulfonate, which are useful intermediates for ketoconazole, are efficiently produced from the obtained optically active form (A).

In addition, optically active trans-(2-aryl-2-halomethyl-1,3-dioxolan-4-yl)methyl carboxylate is isomerized to a cis form thereof in the presence of an acid catalyst.

EP 2 325 178 A1

## Description

Technical Field

The present invention relates to a production method of an optically active compound, an isomerization method of an optically active compound and an optically active compound obtained by the methods.

Background Art

Ketoconazole is widely used as an antifungal agent, and is a highly useful compound. Thus, some synthesis methods have been reported.
As one of them, a method including synthesizing optically active (2-aryl-2-halomethyl-1,3-dioxolan-4-yl)methyl sulfonate, which is a useful intermediate, by ketal exchange between an optically active solketal derivative and aryl halomethyl ketone, and leading the sulfonate to ketoconazole is known. However, solketal is associated with the problems of extremely high cost, poor availability, and low yield in the conversion step to a useful intermediate (see non-patent document 1).

On the other hand, a method including synthesizing optically active (2-aryl-2-halomethyl-1,3-dioxolan-4-yl) methyl calboxylate, which is a useful intermediate, from easily available optically active epichlorohydrin, and leading the calboxylate to ketoconazole has also been reported. However, the method is associated with the problems of low yields and decreased optical purity in the reaction between epichlorohydrin and aryl halomethyl ketone.
Therefore, it was necessary to synthesize 2-aryl-2-halomethyl-4-chloromethyl-1,3-dioxolane by performing ketal exchange with aryl halomethyl ketone after once leading epichlorohydrin to chloromethylacetonide, and then lead the compound to optically active (2-aryl-2-halomethyl-1,3-dioxolan-4-yl)methyl carboxylate. However, a decrease in the optical purity cannot be prevented even by this method, and therefore, the optical purity needs to be increased in the steps up to the final induction to ketoconazole (see non-patent document 2).

Among the optically active (2-aryl-2-halomethyl-1,3-dioxolan-4-yl)methyl carboxylates, a cis form is useful as an intermediate for ketoconazole and the like. According to the existing methods, however, since a cis form and a trans form of optically active (2-aryl-2-halomethyl-1,3-dioxolan-4-yl)methyl carboxylate are produced almost at a ratio of 1:1, the cis form needs to be separated and purified from a mixture thereof by recrystallization, thus defectively wasting the trans form (see non-patent document 2).

For effective utilization of unnecessary optically active trans-(2-aryl-2-halomethyl-1,3-dioxolan-4-yl)methyl carboxylate, optically active trans-(2-aryl-2-halomethyl-1,3-dioxolan-4-yl)methyl carboxylate may be subjected to acid hydrolysis to give aryl(halomethyl)ketone, from which optically active cis-(2-aryl-2-halomethyl-1,3-dioxolan-4-yl)methyl carboxylate may be synthesized again by the above-mentioned method (non-patent document 2). However, such method shows low efficiency and is uneconomical. Therefore, a trans form is ideally directly converted (isomerized) to a cis form.
non-patent document 1: Rotstein, D.M. et al., Journal of Medicinal Chemistry, 1992, 35, p.2818-2825.
non-patent document 2: Camps, P. et al., Tetrahedron Asymmetry, 1995, 6, p.1283-1294.

Disclosure of the Invention

Problems to be Solved by the Invention

An object of the present invention is to provide a method of constructing a 2-aryl-2-halomethyl-1,3-dioxolane ring with ease without decreasing the optical purity, and leading to optically active (2-aryl-2-halomethyl-1,3-dioxolan-4-yl)methyl carboxylate and optically active (2-aryl-2-halomethyl-1,3-dioxolan-4-yl)methyl sulfonate, which are useful intermediate for ketoconazole and the like.
Another object of the present invention is to provide a method of efficiently converting (isomerizing) unnecessary optically active trans-(2-aryl-2-halomethyl-1,3-dioxolan-4-yl)methyl carboxylate to optically active cis-(2-aryl-2-halomethyl-1,3-dioxolan-4-yl)methyl carboxylate useful as an intermediate for ketoconazole and the like.

Means of Solving the Problems

The present inventors have conducted intensive studies in view of the aforementioned problems and unexpectedly found that 2-aryl-2-halomethyl-4-chloromethyl-1,3-dioxolane can be synthesized easily and in a high yield without decreasing the optical purity by reacting easily available, economical optically active monochlorohydrin with aryl (halomethyl)ketone in an acid catalyst. Furthermore, they have developed a method of leading said compound to optically active (2-aryl-2-halomethyl-1,3-dioxolan-4-yl)methyl carboxylate and optically active (2-aryl-2-halomethyl-1,3-dioxolan-4-yl)methyl sulfonate, which are useful intermediate for ketoconazole and the like.

In addition, they have found that optically active trans-(2-aryl-2-halomethyl-1,3-dioxolan-4-yl)methyl carboxylate can be easily isomerized to optically active cis-(2-aryl-2-halomethyl-1,3-dioxolan-4-yl)methyl carboxylate in the presence of an acid catalyst, which resulted in the completion of the present invention.

Accordingly, the present invention includes the following [1] to [46].

[1] A method of producing (2R,trans)-2-aryl-2-halomethyl-4-chloromethyl-1,3-dioxolane represented by the formula [III]

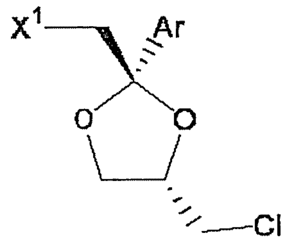

[III]

wherein Ar is a substituted or unsubstituted aromatic ring group, or a substituted or unsubstituted heteroaromatic ring group, and $X^1$ is a halogen atom, and/or (2S,cis)-2-aryl-2-halomethyl-4-chloromethyl-1,3-dioxolane represented by the formula [IV]

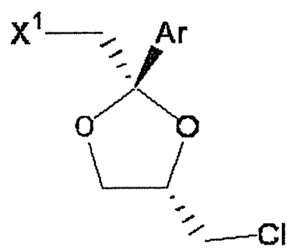

[IV]

wherein each symbol is as defined above, comprising reacting aryl(halomethyl)ketone represented by the formula [I]

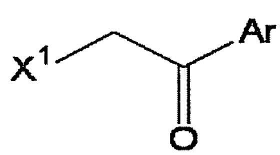

[I]

wherein each symbol is as defined above, with (S)-monochlorohydrin represented by the formula [II]

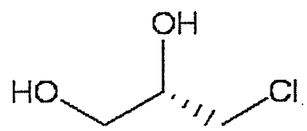

[II]

in the presence of an acid catalyst. [2] The production method of the above-mentioned [1], wherein $X^1$ is a halogen atom other than a chlorine atom, and (S)-monochlorohydrin represented by the formula [II] is added to aryl (halomethyl)ketone represented by the formula [I].

[3] The production method of the above-mentioned [2], wherein (S)-monochlorohydrin is added such that the abundance of (S)-monochlorohydrin represented by the formula [II] during the reaction is not more than 0.2 equivalent of the amount used of aryl(halomethyl)ketone represented by the formula [I].
[4] The production method of the above-mentioned [2] or [3], wherein (S)-monochlorohydrin is added such that the abundance of (S)-monochlorohydrin represented by the formula [II] during the reaction is not more than 0.1 equivalent of the amount used of aryl(halomethyl)ketone represented by the formula [I].
[5] The production method of any of the above-mentioned [1] to [4], wherein an acid catalyst that does not extricate a halide ion other than $X^1$ is used as the acid catalyst.
[6] A method of producing (2R,trans)-(2-aryl-2-halomethyl-1,3-dioxolan-4-yl)methyl carboxylate represented by the formula [V]
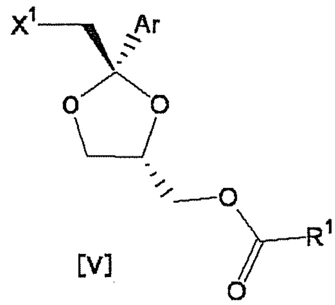

[V]

wherein Ar is a substituted or unsubstituted aromatic ring group, or a substituted or unsubstituted heteroaromatic ring group, $X^1$ is a halogen atom, and $R^1$ is a substituted or unsubstituted hydrocarbon group, or a substituted or unsubstituted aromatic ring group, and/or (2S,cis)-(2-aryl-2-halomethyl-1,3-dioxolan-4-yl)methyl carboxylate represented by the formula [VI]
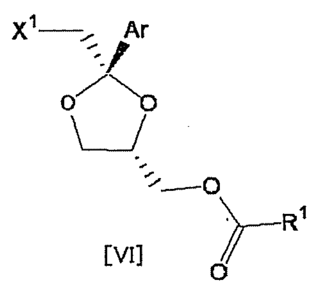

[VI]

wherein each symbol is as defined above, comprising converting (2R,trans)-2-aryl-2-halomethyl-4-chloromethyl-1,3-dioxolane represented by the formula [III] and/or (2S,cis)-2-aryl-2-halomethyl-4-chloromethyl-1,3-dioxolane represented by the formula [IV] obtained by the production method of any of the above-mentioned [1] to [5] to (2R,trans)-(2-aryl-2-halomethyl-1,3-dioxolan-4-yl)methyl carboxylate and/or (2S,cis)-(2-aryl-2-halomethyl-1,3-dioxolan-4-yl)methyl carboxylate.

[7] A method of producing (2S,cis)-(2-aryl-2-halomethyl-1,3-dioxolan-4-yl)methyl sulfonate represented by the formula [X]

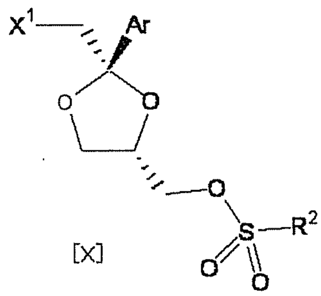

[X]

wherein Ar is a substituted or unsubstituted aromatic ring group, or a substituted or unsubstituted heteroaromatic ring group, $X^1$ is a halogen atom, and $R^2$ is a substituted or unsubstituted hydrocarbon group, or a substituted or unsubstituted aromatic ring group, comprising a step of hydrolyzing (2R,trans)-(2-aryl-2-halomethyl-1,3-dioxolan-4-yl) methyl carboxylate represented by the formula [V] and/or (2S,cis)-(2-aryl-2-halomethyl-1,3-dioxolan-4-yl)methyl carboxylate represented by the formula [VI], which are obtained by the production method of the above-mentioned [6] to give (2R,trans)-2-aryl-2-halomethyl-1,3-dioxolan-4-methanol represented by the formula [VII]

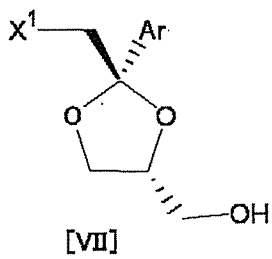

[VII]

wherein each symbol is as defined above, and/or (2S,cis)-2-aryl-2-halomethyl-1,3-dioxolan-4-methanol represented by the formula [VIII]

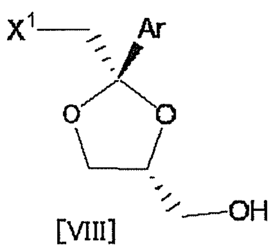

[VIII]

wherein each symbol is as defined above,

a step of converting (2R,trans)-2-aryl-2-halomethyl-1,3-dioxolan-4-methanol represented by the formula [VII] and/or (2S,cis)-2-aryl-2-halomethyl-1,3-dioxolan-4-methanol represented by the formula [VIII] to (2R,trans)-(2-aryl-2-halomethyl-1,3-dioxolan-4-yl)methyl sulfonate represented by the formula [IX]

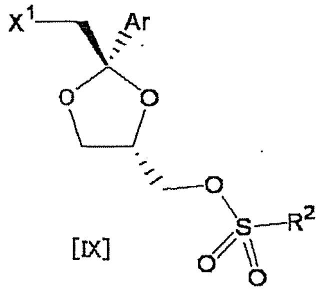

[IX]

wherein each symbol is as defined above, and/or (2S,cis)-(2-aryl-2-halomethyl-1,3-dioxolan-4-yl)methyl sulfonate represented by the formula [X], and

a step of isolating (2S,cis)-(2-aryl-2-halomethyl-1,3-dioxolan-4-yl)methyl sulfonate represented by the formula [X] ) by recrystallization from a mixture containing (2R,trans)-(2-aryl-2-halomethyl-1,3-dioxolan-4-yl)methyl sulfonate represented by the formula [IX] and (2S,cis)-(2-aryl-2-halomethyl-1,3-dioxolan-4-yl)methyl sulfonate represented by the formula [X].

[8] A method of producing (2S,cis)-(2-aryl-2-halomethyl-1,3-dioxolan-4-yl)methyl carboxylate represented by the formula [VI], wherein (2S,cis)-(2-aryl-2-halomethyl-1,3-dioxolan-4-yl)methyl carboxylate represented by the formula [VI] is isolated by recrystallization from a mixture containing (2R,trans)-(2-aryl-2-halomethyl-1,3-dioxolan-4-yl)methyl carboxylate represented by the formula [V] and (2S,cis)-(2-aryl-2-halomethyl-1,3-dioxolan-4-yl)methyl carboxylate represented by the formula [VI], which are obtained by the production method of the above-mentioned [6].

[9] A method of producing (2S,cis)-(2-aryl-2-halomethyl-1,3-dioxolan-4-yl)methyl carboxylate represented by the formula [VI], comprising isomerizing (2R,trans)-(2-aryl-2-halomethyl-1,3-dioxolan-4-yl)methyl carboxylate represented by the formula [V], which is contained in the mother liquid of the recrystallization in the method of the above-mentioned [8], in the presence of an acid catalyst.

[10] The production method of the above-mentioned [9], which is performed in at least one kind of solvent selected from aliphatic hydrocarbon solvents, aromatic hydrocarbon solvents, halogenated hydrocarbon solvents, nitrile solvents, ester solvents and acid catalysts.

[11] The production method of the above-mentioned [9] or [10], comprising a step of isolating (2S,cis)-(2-aryl-2-halomethyl-1,3-dioxolan-4-yl)methyl carboxylate represented by the formula [VI] after the isomerization step.

[12] The production method of the above-mentioned [11], comprising isolation by recrystallization.

[13] A method of producing (2S,cis)-(2-aryl-2-halomethyl-1,3-dioxolan-4-yl)methyl carboxylate represented by the formula [VI], comprising reacting a mixture containing (2R,trans)-(2-aryl-2-halomethyl-1,3-dioxolan-4-yl)methyl carboxylate represented by the formula [V] and (2S,cis)-(2-aryl-2-halomethyl-1,3-dioxolan-4-yl)methyl carboxylate represented by the formula [VI], which are obtained by the production method of the above-mentioned [6], in the presence of an acid catalyst to isomerize (2R,trans)-(2-aryl-2-halomethyl-1,3-dioxolan-4-yl)methyl carboxylate represented by the formula [V] to (2S,cis)-(2-aryl-2-halomethyl-1,3-dioxolan-4-yl)methyl carboxylate represented by the formula [VI], while simultaneously precipitating (2S,cis)-(2-aryl-2-halomethyl-1,3-dioxolan-4-yl)methyl carboxylate represented by the formula [VI] as crystals.

[14] The production method of the above-mentioned [13], which is performed in at least one kind of solvent selected from aliphatic hydrocarbon solvents, aromatic hydrocarbon solvents, halogenated hydrocarbon solvents, nitrile solvents, ester solvents and acid catalysts.

[15] The production method of any of the above-mentioned [9] to [14], wherein the acid catalyst is a Lewis acid or an organic acid.

[16] A method of producing (2S,cis)-(2-aryl-2-halomethyl-1,3-dioxolan-4-yl)methyl sulfonate represented by the formula [X]

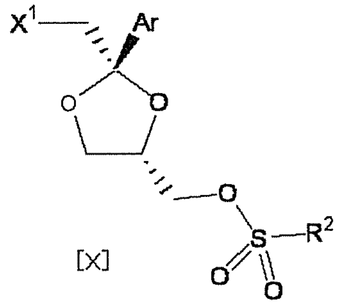

[X]

wherein Ar is a substituted or unsubstituted aromatic ring group, or a substituted or unsubstituted heteroaromatic ring group, and $X^1$ is a halogen atom,
comprising a step of hydrolyzing (2S,cis)-(2-aryl-2-halomethyl-1,3-dioxolan-4-yl)methyl carboxylate represented by the formula [VI] obtained by the production method of any of the above-mentioned [8] to [15] to give (2S,cis)-2-aryl-2-halomethyl-1,3-dioxolan-4-methanol represented by the formula [VIII]
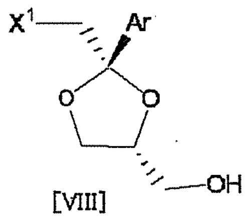

[VIII]

wherein each symbol is as defined above, and a step of converting (2S,cis)-2-aryl-2-halomethyl-1,3-dioxolan-4-methanol represented by the formula [VIII] to (2S,cis)-(2-aryl-2-halomethyl-1,3-dioxolan-4-yl)methyl sulfonate represented by the formula [X].
[17] A method of producing (2S,trans)-2-aryl-2-halomethyl-4-chloromethyl-1,3-dioxolane represented by the formula [XII]
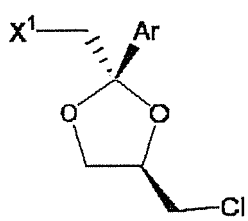

[XII]

wherein Ar is a substituted or unsubstituted aromatic ring group, or a substituted or unsubstituted heteroaromatic ring group, and $X^1$ is a halogen atom, and/or (2R,cis)-2-aryl-2-halomethyl-4-chloromethyl-1,3-dioxolane represented by the formula [XIII]
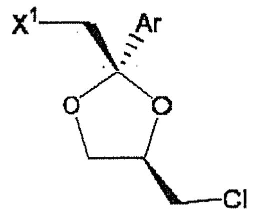

[XIII]

wherein each symbol is as defined above, comprising reacting aryl(halomethyl)ketone represented by the formula [I]

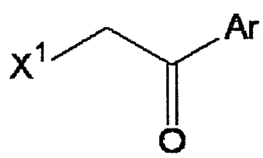

[I]

wherein each symbol is as defined above, with (R)-monochlorohydrin represented by the formula [XI]

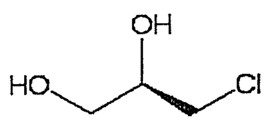

[XI]

in the presence of an acid catalyst.

[18] The production method of the above-mentioned [17], wherein $X^1$ is a halogen atom other than a chlorine atom, and (R)-monochlorohydrin represented by the formula [XI] is added to aryl(halomethyl)ketone represented by the formula [I].

[19] The production method of the above-mentioned [18], wherein (R)-monochlorohydrin is added such that the abundance of (R)-monochlorohydrin represented by the formula [XI] during the reaction is not more than 0.2 equivalent of the amount used of aryl(halomethyl)ketone represented by the formula [I].

[20] The production method of the above-mentioned [18] or [19], wherein (R)-monochlorohydrin is added such that the abundance of (R)-monochlorohydrin represented by the formula [XI] during the reaction is not more than 0.1 equivalent of the amount used of aryl(halomethyl)ketone represented by the formula [I].

[21] The production method of any of the above-mentioned [17] to [20], wherein an acid catalyst that does not extricate a halide ion other than $X^1$ is used as the acid catalyst.

[22] A method of producing (2S,trans)-(2-aryl-2-halomethyl-1,3-dioxolan-4-yl)methyl carboxylate represented by the formula [XIV]

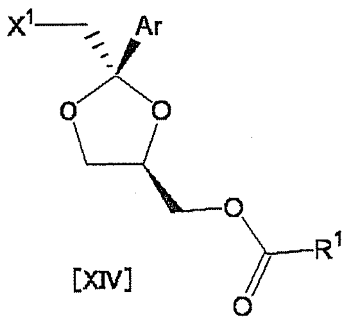

[XIV]

wherein Ar is a substituted or unsubstituted aromatic ring group, or a substituted or unsubstituted heteroaromatic ring group, $X^1$ is a halogen atom, and $R^1$ is a substituted or unsubstituted hydrocarbon group, or a substituted or unsubstituted aromatic ring group, and/or (2R,cis)-(2-aryl-2-halomethyl-1,3-dioxolan-4-yl)methyl carboxylate represented by the formula [XV]

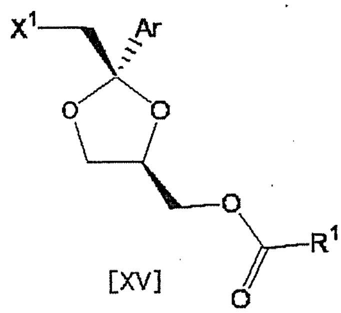

[XV]

wherein each symbol is as defined above, comprising converting (2S,trans)-2-aryl-2-halomethyl-4-chloromethyl-1,3-dioxolane represented by the formula [XII] and/or (2R,cis)-2-aryl-2-halomethyl-4-chloromethyl-1,3-dioxolane, represented by the formula [XIII], which are obtained by the production method of any of the above-mentioned [17] to [21], to (2S,trans)-(2-aryl-2-halomethyl-1,3-dioxolan-4-yl)methyl carboxylate, and/or (2R,cis)-(2-aryl-2-halomethyl-1,3-dioxolan-4-yl)methyl carboxylate.

[23] A method of producing (2R,cis)-(2-aryl-2-halomethyl-1,3-dioxolan-4-yl)methyl sulfonate represented by the formula [XIX]

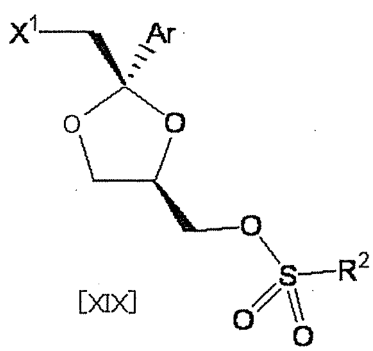

[XIX]

wherein Ar is a substituted or unsubstituted aromatic ring group, or a substituted or unsubstituted heteroaromatic ring group, $X^1$ is a halogen atom, and $R^2$ is a substituted or unsubstituted hydrocarbon group, or a substituted or

unsubstituted aromatic ring group, comprising hydrolyzing (2S,trans)-(2-aryl-2-halomethyl-1,3-dioxolan-4-yl)methyl carboxylate represented by the formula [XIV] and/or (2R,cis)-(2-aryl-2-halomethyl-1,3-dioxolan-4-yl)methyl carboxylate represented by the formula [XV], which are obtained by the production method of the above-mentioned [22], to give (2S,trans)-2-aryl-2-halomethyl-1,3-dioxolan-4-methanol represented by the formula [XVI]

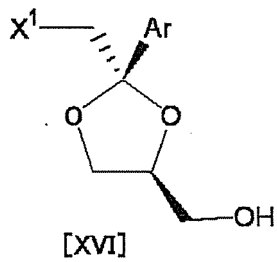

[XVI]

wherein each symbol is as defined above, and/or (2R,cis)-2-aryl-2-halomethyl-1,3-dioxolan-4-methanol represented by the formula [XVII]

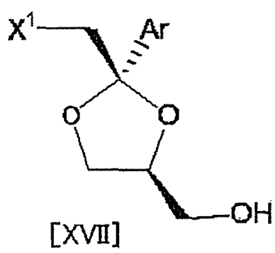

[XVII]

wherein each symbol is as defined above,
a step of converting (2S,trans)-2-aryl-2-halomethyl-1,3-dioxolan-4-methanol represented by the formula [XVI] and/or (2R,cis)-2-aryl-2-halomethyl-1,3-dioxolan-4-methanol represented by the formula [XVII] to (2S,trans)-(2-aryl-2-halomethyl-1,3-dioxolan-4-yl)methyl sulfonate represented by the formula [XVIII]

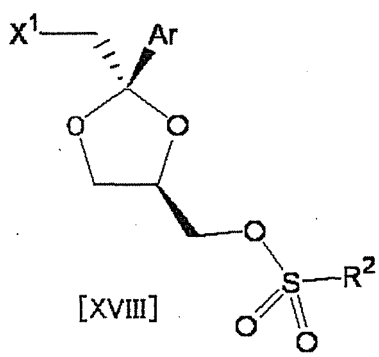

[XVIII]

wherein each symbol is as defined above, and/or (2R,cis)-(2-aryl-2-halomethyl-1,3-dioxolan-4-yl)methyl sulfonate represented by the formula [XIX], and

a step of isolating (2R,cis)-(2-aryl-2-halomethyl-1,3-dioxolan-4-yl)methyl sulfonate represented by the formula [XIX] by recrystallization from a mixture containing (2S,trans)-(2-aryl-2-halomethyl-1,3-dioxolan-4-yl)methyl sulfonate represented by the formula [XVIII] and (2R,cis)-(2-aryl-2-halomethyl-1,3-dioxolan-4-yl)methyl sulfonate represented by

the formula [XIX].

[24] A method of producing (2R,cis)-(2-aryl-2-halomethyl-1,3-dioxolan-4-yl)methyl carboxylate represented by the formula [XV], comprising isolating (2R,cis)-(2-aryl-2-halomethyl-1,3-dioxolan-4-yl)methyl carboxylate represented by the formula [XV] by crystallization from a mixture containing (2S,trans)-(2-aryl-2-halomethyl-1,3-dioxolan-4-yl)methyl carboxylate represented by the formula [XIV] and (2R,cis)-(2-aryl-2-halomethyl-1,3-dioxolan-4-yl)methyl carboxylate represented by the formula [XV], which are obtained by the production method of the above-mentioned [22].

[25] A method of producing (2R,cis)-(2-aryl-2-halomethyl-1,3-dioxolan-4-yl)methyl carboxylate represented by the formula [XV], comprising isomerizing (2S,trans)-(2-aryl-2-halomethyl-1,3-dioxolan-4-yl)methyl carboxylate represented by the formula [XIV], which is contained in the mother liquid of the recrystallization in the method of the above-mentioned [24], in the presence of an acid catalyst.

[26] The production method of the above-mentioned [25], which is performed in at least one kind of solvent selected from aliphatic hydrocarbon solvents, aromatic hydrocarbon solvents halogenated hydrocarbon solvents, nitrile solvents, ester solvents and acid catalysts.

[27] The production method of the above-mentioned [25] or [26], comprising a step of isolating (2R,cis)-(2-aryl-2-halomethyl-1,3-dioxolan-4-yl)methyl carboxylate represented by the formula [XV] after the isomerization step.

[28] The production method of the above-mentioned [27], comprising isolation by recrystallization.

[29] A method of producing (2R,cis)-(2-aryl-2-halomethyl-1,3-dioxolan-4-yl)methyl carboxylate represented by the formula [XV], comprising reacting a mixture containing (2S,trans)-(2-aryl-2-halomethyl-1,3-dioxolan-4-yl)methyl carboxylate represented by the formula [XIV] and (2R,cis)-(2-aryl-2-halomethyl-1,3-dioxolan-4-yl)methyl carboxylate represented by the formula [XV], which are obtained by the production method of the above-mentioned [22], in the presence of an acid catalyst to isomerize (2S,trans)-(2-aryl-2-halomethyl-1,3-dioxolan-4-yl)methyl carboxylate represented by the formula [XIV] to (2R,cis)-(2-aryl-2-halomethyl-1,3-dioxolan-4-yl)methyl carboxylate represented by the formula [XV], while simultaneously precipitating (2R,cis)-(2-aryl-2-halomethyl-1,3-dioxolan-4-yl)methyl carboxylate represented by the formula [XV] as crystals.

[30] The production method of the above-mentioned [29], which is performed in at least one kind of solvent selected from aliphatic hydrocarbon solvents, aromatic hydrocarbon solvents, halogenated hydrocarbon solvents, nitrile solvents, ester solvents and acid catalysts.

[31] The production method of any of the above-mentioned [25] to [30], wherein the acid catalyst is a Lewis acid or an organic acid.

[32] A method of producing (2R,cis)-(2-aryl-2-halomethyl-1,3-dioxolan-4-yl)methyl sulfonate represented by the formula [XIX]

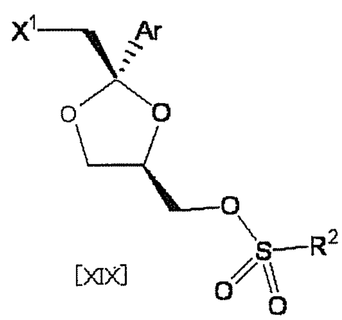

[XIX]

wherein Ar is a substituted or unsubstituted aromatic ring group, or a substituted or unsubstituted heteroaromatic ring group, and $X^1$ is a halogen atom, comprising a step of hydrolyzing (2R,cis)-(2-aryl-2-halomethyl-1,3-dioxolan-4-yl)methyl carboxylate represented by the formula [XV], which is obtained by the production method of any of the above-mentioned [24] to [31], to give (2R,cis)-2-aryl-2-halomethyl-1,3-dioxolan-4-methanol represented by the formula [XVII]

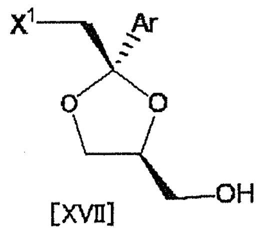

[XVII]

wherein each symbol is as defined above, and a step of converting (2R,cis)-2-aryl-2-halomethyl-1,3-dioxolan-4-methanol represented by the formula [XVII] to (2R,cis)-(2-aryl-2-halomethyl-1,3-dioxolan-4-yl)methyl sulfonate represented by the formula [XIX].

[33] The production method of any of the above-mentioned [1] to [32], wherein aryl(halomethyl)ketone represented by the formula [I] is (4-chlorophenyl)-2-bromomethylketone, (2,4-dichlorophenyl)-2-bromomethylketone, (4-fluorophenyl)-2-bromomethylketone, (2,4-difluorophenyl)-2-bromomethylketone, (4-chloro-2-trifluoromethylphenyl)-2-bromomethylketone, phenyl-2-bromomethylketone, (4-methylphenyl)-2-bromomethylketone, (4-biphenyl)-2-bromomethylketone, (2-naphthyl)-2-bromomethylketone, (4-methoxyphenyl)-2-bromomethylketone, (4-phenoxyphenyl)-2-bromomethylketone, (4-nitrophenyl)-2-bromomethylketone, (2-thienyl)-2-bromomethylketone, (4-chlorophenyl)-2-chloromethylketone, phenyl-2-chloromethylketone or (4-methylphenyl)-2-chloromethylketone.

[34] A method of producing (2S,cis)-(2-aryl-2-halomethyl-1,3-dioxolan-4-yl)methyl carboxylate represented by the formula [VI]

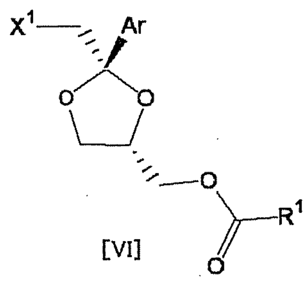

[VI]

wherein Ar is a substituted or unsubstituted aromatic ring group, or a substituted or unsubstituted heteroaromatic ring group, $X^1$ is a halogen atom, and $R^1$ is a substituted or unsubstituted hydrocarbon group, or a substituted or unsubstituted aromatic ring group, comprising a step of isomerizing (2R,trans)-(2-aryl-2-halomethyl-1,3-dioxolan-4-yl) methyl carboxylate represented by the formula [V]

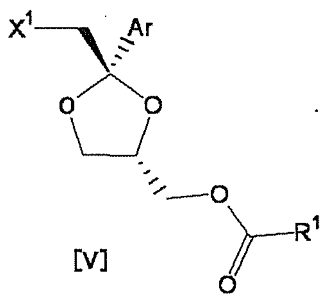

[V]

wherein each symbol is as defined above, to (2S,cis)-(2-aryl-2-halomethyl-1,3-dioxolan-4-yl)methyl carboxylate represented by the formula [VI], in the presence of an acid catalyst.

[35] A method of producing (2R,cis)-(2-aryl-2-halomethyl-1,3-dioxolan-4-yl)methyl carboxylate represented by the formula [XV]

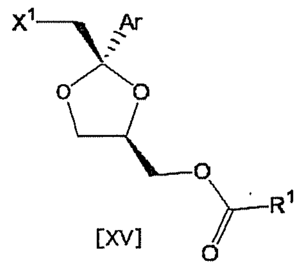

[XV]

wherein Ar is a substituted or unsubstituted aromatic ring group, or a substituted or unsubstituted heteroaromatic ring group, $X^1$ is a halogen atom, and $R^1$ is a substituted or unsubstituted hydrocarbon group, or a substituted or unsubstituted aromatic ring group, comprising a step of isomerizing (2S,trans)-(2-aryl-2-halomethyl-1,3-dioxolan-4-yl)methyl carboxylate represented by the formula [XIV]

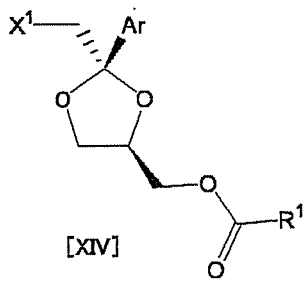

[XIV]

wherein each symbol is as defined above, to (2R,cis)-(2-aryl-2-halomethyl-1,3-dioxolan-4-yl)methyl carboxylate represented by the formula [XV], in the presence of an acid catalyst.

[36] The production method of the above-mentioned [34] or [35], wherein, in the optically active (2-aryl-2-halomethyl-1,3-dioxolan-4-yl)methyl carboxylates represented by the formulas [V], [VI], [XIV] and [XV], Ar is an aromatic ring group or a halogen-substituted aromatic ring group, and $X^1$ is a chlorine atom or a bromine atom.

[37] The production method of any of the above-mentioned [34] to [36], which is performed in at least one kind of solvent selected from aliphatic hydrocarbon solvents, aromatic hydrocarbon solvents, halogenated hydrocarbon solvents, nitrile solvents, ester solvents and acid catalysts.

[38] The production method of any of the above-mentioned [34] to [37], wherein the acid catalyst is a Lewis acid or an organic acid.

[39] The production method of any of the above-mentioned [34] to [38], comprising a step of isolating cis-(2-aryl-2-halomethyl-1,3-dioxolan-4-yl)methyl carboxylate after the isomerization step.

[40] The production method of any of the above-mentioned [34] to [39], comprising isolation by recrystallization.

[41] The production method of any of the above-mentioned [34] to [40], wherein, in the isomerization step, isomerization is performed while precipitating cis-(2-aryl-2-halomethyl-1,3-dioxolan-4-yl)methyl carboxylate as crystals.

[42] The production method of any of the above-mentioned [34] to [41], comprising a step of quenching the acid catalyst with a base after completion of the reaction.

[43] A (2S,cis)-(2-aryl-2-halomethyl-1,3-dioxolan-4-yl)methyl carboxylate represented by the formula [VI]

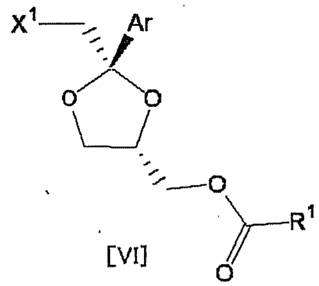

[VI]

wherein Ar is a substituted or unsubstituted aromatic ring group, or a substituted or unsubstituted heteroaromatic ring group, $X^1$ is a halogen atom, and $R^1$ is a substituted or unsubstituted hydrocarbon group, or a substituted or unsubstituted aromatic ring group, having a content of a halogen-exchanged compound wherein $X^1$ is exchanged with other halogen group of not more than 0.2%.

[44] A (2R,cis)-(2-aryl-2-halomethyl-1,3-dioxolan-4-yl)methyl carboxylate represented by the formula [XV]

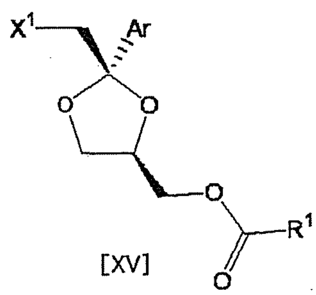

[XV]

wherein Ar is a substituted or unsubstituted aromatic ring group, or a substituted or unsubstituted heteroaromatic ring group, $X^1$ is a halogen atom, and $R^1$ is a substituted or unsubstituted hydrocarbon group, or a substituted or unsubstituted aromatic ring group, having a content of a halogen-exchanged compound wherein $X^1$ is exchanged with other halogen group of not more than 0.2%.

[45] The compound of the above-mentioned [43] or [44], wherein $X^1$ is a halogen atom other than a chlorine atom, and the content of the halogen-exchanged compound, wherein $X^1$ is a chlorine atom, is not more than 0.2%.

[46] The compound of the above-mentioned [43] or [44], wherein $X^1$ is a bromine atom, Ar is 4-chlorophenyl, 2,4-dichlorophenyl, 4-fluorophenyl, 2,4-difluorophenyl, 4-chloro-2-trifluoromethylphenyl, phenyl, 4-methylphenyl, 4-biphenyl, 2-naphthyl, 4-methoxyphenyl, 4-phenoxyphenyl, 4-nitrophenyl or 2-thienyl, $R^1$ is phenyl, methyl, ethyl, 4-nitrophenyl, 4-methoxyphenyl, 4-chlorophenyl or heptyl, and the content of the halogen-exchanged compound, wherein $X^1$ is a chlorine atom, is not more than 0.2%.

Effect of the Invention

Using the production method of the present invention, easily available and economical optically active monochlorohydrin used as a starting material is reacted with aryl(halomethyl)ketone, and optically active 2-aryl-2-halomethyl-4-chloromethyl-1,3-dioxolane can be produced efficiently while suppressing a decrease in the optical purity. In addition, optically active (2-aryl-2-halomethyl-1,3-dioxolan-4-yl)methyl carboxylate and optically active (2-aryl-2-halomethyl-1,3-dioxolan-4-yl)methyl sulfonate, which are useful as intermediates for ketoconazole can be efficiently produced from the optically active 2-aryl-2-halomethyl-4-chloromethyl-1,3-dioxolane without decreasing the optical purity.
In addition, using the isomerization method of the present invention, unnecessary optically active trans-(2-aryl-2-halomethyl-1,3-dioxolan-4-yl)methyl carboxylate can be efficiently converted to optically active cis-(2-aryl-2-halomethyl-1,3-dioxolan-4-yl)methyl carboxylate useful as an intermediate for ketoconazole.

Best Description of Embodiments

A detailed explanation is given below.

Starting material

The starting materials are aryl(halomethyl)ketone and optically active monochlorohydrin. Aryl(halomethyl)ketone which is one of the starting materials is represented by the formula [I].

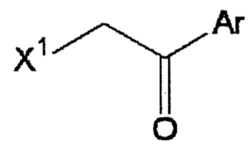

[I]

wherein Ar is a substituted or unsubstituted aromatic ring group, or a substituted or unsubstituted heteroaromatic ring group, and $X^1$ is a halogen atom.

In aryl(halomethyl)ketone represented by the formula [I], Ar is a substituted or unsubstituted aromatic ring group, or a substituted or unsubstituted heteroaromatic ring group.

Examples of the aromatic ring group include an aryl group having a carbon number of 6 to 14 (e.g., phenyl group, naphthyl group etc.).

Examples of the heteroaromatic ring group include a 5- or 6-membered heteroaromatic ring group containing 1 to 3 hetero atoms selected from an oxygen atom, a nitrogen atom and a sulfur atom (e.g., furyl group, thienyl group, pyrrolyl group, imidazolyl group, pyrazolyl group, oxazolyl group, isoxazolyl group, thiazolyl group, isothiazolyl group, triazolyl group, oxadiazolyl group, thiadiazolyl group, pyridyl group, pyrimidinyl group, pyrazinyl group, pyridazinyl group, triazinyl group etc.).

Examples of the atom and substituent that can substitute the aromatic ring group and the heteroaromatic ring group include alkyl groups having a carbon number of 1 to 8 such as methyl group, ethyl group and the like, haloalkyl groups having a carbon number of 1 to 8 such as trifluoromethyl group and the like, aryl groups having a carbon number of 6 to 14 such as phenyl group and the like, aralkyl groups having a carbon number of 7 to 15 such as benzyl group and the like, unsaturated hydrocarbon groups having a carbon number of 2 to 8 such as vinyl group and the like (alkenyl group having a carbon number of 2 to 8, alkynyl group having a carbon number of 2 to 8), halogen atoms such as fluorine atom, chlorine atom, bromine atom and the like, alkoxy groups having a carbon number of 1 to 8 (e.g., methoxy group, ethoxy group etc.), carbonyloxy groups having a carbon number of 2 to 8 (e.g., acetyloxy group, propionyloxy group etc.), ether group (e.g., aryloxy group having a carbon number of 6 to 14 such as phenoxy group and the like), thioether groups (e.g., alkylthio group having a carbon number of 1 to 8 such as methylthio group, ethylthio group and the like), carboxy group, alkoxycarbonyl groups having a carbon number of 2 to 8 (e.g., methoxycarbonyl group, ethoxycarbonyl group etc.), formyl group, alkylcarbonyl groups having a carbon number of 2 to 8 (e.g., acetyl group, propionyl group etc.), amino groups (e.g., amino group, mono $C_{1-8}$ alkylamino group, di $C_{1-8}$ alkylamino group etc.), nitro group, aminocarbonyl groups (e.g., aminocarbonyl group, $C_{1-8}$ alkylaminocarbonyl group etc.), carbonylamino groups (e.g., carbonylamino group, $C_{1-8}$ alkylcarbonylamino group), silyl groups (tri $C_{1-8}$ alkylsilyl group such as trimethylsilyl group and the like, etc.), phosphine groups (di $C_{1-8}$ alkylphosphine group such as di n-butylphosphine group and the like, di $C_{6-10}$ arylphosphine group such as diphenylphosphine group and the like, etc.), sulfonyl groups (e.g., $C_{1-8}$ alkylsulfonyl groups such as methanesulfonyl group and the like) and the like.

Examples of the halogen atom represented by $X^1$ include a fluorine atom, a chlorine atom, a bromine atom and an iodine atom.

The aryl(halomethyl)ketone represented by the formula [I] can be prepared by known Friedel-Crafts reaction, and commercially available products can also be used.

Preferable specific examples of aryl(halomethyl)ketone represented by the formula [I] include (4-chlorophenyl)-2-bromomethylketone, (2,4-dichlorophenyl)-2-bromomethylketone, (4-fluorophenyl)-2-bromomethylketone, (2,4-difluorophenyl)-2-bromomethylketone, (4-chloro-2-trifluoromethylphenyl)-2-bromomethylketone, phenyl-2-bromomethylketone, (4-methylphenyl)-2-bromomethylketone, (4-biphenyl)-2-bromomethylketone, (2-naphthyl)-2-bromomethylketone, (4-methoxyphenyl)-2-bromomethylketone, (4-phenoxyphenyl)-2-bromomethylketone, (4-nitrophenyl)-2-bromomethylketone, (2-thienyl)-2-bromomethylketone, (4-chlorophenyl)-2-chloromethylketone, phenyl-2-chloromethylketone and (4-methylphenyl)-2-chloromethylketone.

The optically active (S)-monochlorohydrin which is one of the starting materials is represented by the formula [II].

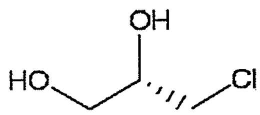

[II]

The optical purity of optically active (S)-monochlorohydrin represented by the formula [II] may be lower than 100%ee, since the optical purity can be increased to a certain level during recrystallization of the object (2-aryl-2-halomethyl-1,3-dioxolan-4-yl)methyl sulfonate and (2-aryl-2-halomethyl-1,3-dioxolan-4-yl)methyl carboxylate. Preferred is not less than 90%ee and more preferred is not less than 95%ee. Using (S)-monochlorohydrin with this level of purity, (2-aryl-2-halomethyl-1,3-dioxolan-4-yl)methyl sulfonate and (2-aryl-2-halomethyl-1,3-dioxolan-4-yl)methyl carboxylate having high optical purity can be effectively produced.

The optically active (R)-monochlorohydrin which is one of the starting materials is represented by the formula [XI].

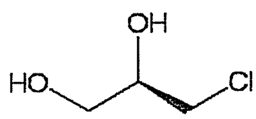

[XI]

The optical purity of optically active (R)-monochlorohydrin represented by the formula [XI] may be lower than 100%ee, since the optical purity can be increased to a certain level during recrystallization of the object (2-aryl-2-halomethyl-1,3-dioxolan-4-yl)methyl sulfonate and (2-aryl-2-halomethyl-1,3-dioxolan-4-yl)methyl carboxylate. Preferred is not less than 90%ee and more preferred is not less than 95%ee. Using (R)-monochlorohydrin with this level of purity, (2-aryl-2-halomethyl-1,3-dioxolan-4-yl)methyl sulfonate and (2-aryl-2-halomethyl-1,3-dioxolan-4-yl)methyl carboxylate having high optical purity can be effectively produced.

The optically active (S)-monochlorohydrin represented by the formula [II] and optically active (R)-monochlorohydrin represented by the formula [XI] can be produced by a known method (Jacobsen, E.N., et. al, Science, 1997, 277, 936-938), and economical commercially available products can also be used.

Each step of the method of the present invention is explained in detail in the following.

The reaction mixture obtained in each step can be separated and purified by a known isolation and purification means (crystallization, recrystallization, column chromatography etc.) after workup (extraction, washing, concentration etc.). In addition, the mixture as a crude product can also be subjected to the next step, without performing isolation and purification.

<u>(1) Synthesis of optically active 2-aryl-2-halomethyl-4-chloromethyl-1,3-dioxolane</u>

The optically active 2-aryl-2-halomethyl-4-chloromethyl-1,3-dioxolane can be synthesized by reacting aryl (halomethyl)ketone with optically active monochlorohydrin in the presence of an acid catalyst.

The amount of optically active monochlorohydrin to be used is preferably 0.3 to 10 equivalents, more preferably 0.5 to 3 equivalents, relative to aryl(halomethyl)ketone.

This reaction affords a mixture of a trans form and a cis form of optically active 2-aryl-2-halomethyl-4-chloromethyl-1,3-dioxolane, wherein the cis form tends to grow somewhat preferentially as compared to the trans form.

As the acid catalyst to be used, organic acids such as toluenesulfonic acid, methanesulfonic acid, acetic acid, trifluoroacetic acid and the like, Lewis acids such as boron trifluoride-diethyl ether complex ($BF_3 \cdot Et_2O$) and the like, inorganic acids such as sulfuric acid, hydrochloric acid, hydrobromic acid etc., and the like can be used.

A solvent is generally used in this step. The kind of the solvent is not particularly limited, and aromatic hydrocarbon solvents such as benzene, toluene and xylene, ethyl acetate, methylene chloride, DMF and the like can be used.
In addition, since a ketalization reaction is an equilibrium reaction, removal of water produced in the system is effective for promoting the progress of the reaction. Thus, azeotropic distillation with a solvent, and use of a dehydrating agent such as molecular sieve and the like are preferable.
The reaction temperature may be about 0˚C to 150˚C, and the reaction time may be generally 1 to 24 hr.

In the synthesis of the optically active 2-aryl-2-halomethyl-4-chloromethyl-1,3-dioxolane wherein the halogen group ($X^1$) of halomethyl of aryl(halomethyl)ketone of the substrate is other than a chlorine atom, when a reaction is performed after mixing the total amounts of aryl(halomethyl)ketone and optically active monochlorohydrin, halogen exchange easily occurs between aryl(halomethyl)ketone and optically active monochlorohydrin in the presence of an acid catalyst to give the corresponding optically active 2-aryl-2-chloromethyl-4-chloromethyl-1,3-dioxolane.
In addition, when an acid catalyst that extricates halide ion ($X^2$) other than $X^1$ is used as the acid catalyst, halogen exchange occurs between halogen atom ($X^2$) of the acid catalyst and halogen group ($X^1$) of aryl(halomethyl)ketone of the substrate, and the corresponding optically active trans-2-aryl-2-halomethyl-4-chloromethyl-1,3-dioxolane represented by the formula [XX]

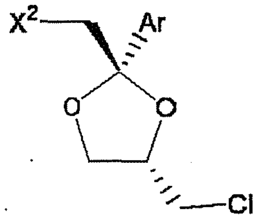

[XX]

wherein $X^2$ is a halogen atom other than $X^1$=$X^2$, and optically active cis-2-aryl-2-halomethyl-4-chloromethyl-1,3-dioxolane represented by the formula [XXI]

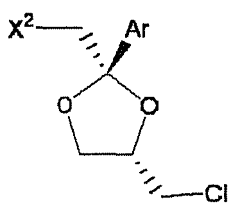

[XXI]

wherein $X^2$ is as defined above, are produced.
Specific examples of the acid catalyst that extricates halide ion ($X^2$) include inorganic acid containing $X^2$ such as $HX^2$, $HX^2O$, $HX^2O_2$, $HX^2O_4$ and the like, and Lewis acids containing $X^2$ such as $ZnX^2{}_2$, $FeX^2{}_3$, $AlX^2{}_3$, $SnX^2{}_4$, $TiX^2{}_4$ and the like excluding $BF_3$.
These halogen-exchanged compounds are difficult to remove even by the below-mentioned recrystallization of (2-aryl-2-halomethyl-1,3-dioxolan-4-yl)methyl sulfonate and (2-aryl-2-halomethyl-1,3-dioxolan-4-yl)methyl carboxylate.
These halogen-exchanged compounds have low reactivity, and are associated with the problems of decrease in yield and the like during induction to ketoconazole,.

(2) Synthesis of optically active 2-aryl-2-halomethyl-4-chloromethyl-1,3-dioxolane while suppressing halogen exchange

The halogen exchange between aryl(halomethyl)ketone and optically active monochlorohydrin is considered to occur due to the high substitution reactivity of halogen group ($X^1$) of aryl(halomethyl)ketone, and easy extrication of chloride ion from monochlorohydrin. This halogen-exchange reaction is considered to take place only with aryl(halomethyl)ketone, and is not considered to occur with 2-aryl-2-halomethyl-4-chloromethyl-1,3-dioxolane. Therefore, this halogen exchange is expected to be suppressed by shortening the contact time between aryl(halomethyl)ketone and

monochlorohydrin.
In the synthesis of optically active 2-aryl-2-halomethyl-4-chloromethyl-1,3-dioxolane wherein halogen group ($X^1$) of halomethyl of aryl(halomethyl)ketone is other than a chlorine atom, therefore, the reaction is preferably performed while controlling the abundance of optically active monochlorohydrin to not more than 0.2 equivalent relative to the amount of aryl(halomethyl)ketone to be used, in an attempt to suppress halogen exchange between aryl(halomethyl)ketone and optically active monochlorohydrin, and suppress production of a halogen-exchanged compound. The reaction while controlling the abundance of the optically active monochlorohydrin to not more than 0.2 equivalent becomes possible by adding optically active monochlorohydrin to aryl(halomethyl)ketone while monitoring the reaction by GC and the like as necessary.

While the method of adding the optically active monochlorohydrin is not particularly limited as long as the optically active monochlorohydrin can be added to aryl(halomethyl)ketone in small portions, a method of adding dropwise the optically active monochlorohydrin or a solution thereof is preferable, since the addition rate can be controlled easily.

As for the addition, optically active monochlorohydrin is preferably added such that the abundance thereof present in the reaction solution is not more than 0.2 equivalent, more preferably not more than 0.1 equivalent, relative to the amount of aryl(halomethyl)ketone to be used (amount initially used).
Furthermore, to also suppress halogen exchange that occurs between halogen atom present in the catalyst and aryl (halomethyl)ketone in the substrate, the reaction may be performed using an acid catalyst that does not extricate a halide ion other than $X^1$.

(3) Synthesis of optically active (2-aryl-2-halomethyl-1,3-dioxolan-4-yl)methyl carboxylate

The optically active (2-aryl-2-halomethyl-1,3-dioxolan-4-yl)methyl carboxylate can be synthesized by reacting optically active 2-aryl-2-halomethyl-4-chloromethyl-1,3-dioxolane with carboxylic acid or a salt thereof and the like.

The optically active (2-aryl-2-halomethyl-1,3-dioxolan-4-yl)methyl carboxylate is preferably synthesized under neutral or basic condition since 1,3-dioxolane ring in 2-aryl-2-halomethyl-4-chloromethyl-1,3-dioxolane is decomposed under acidic conditions.
To suppress the decomposition of 1,3-dioxolane ring, a salt of carboxylic acid is preferably used.

Examples of the carboxylic acid to be used include carboxylic acid represented by the formula: $R^1COOH$ wherein $R^1$ is a substituted or unsubstituted hydrocarbon group, or a substituted or unsubstituted aromatic ring group.
Examples of the hydrocarbon group for $R^1$ include an alkyl group having a carbon number of 1 to 8 such as a methyl group, an ethyl group and the like, an aralkyl group having a carbon number of 7 to 11 such as a benzyl group and the like, an unsaturated hydrocarbon group having a carbon number of 2 to 8 such as a vinyl group and the like (an alkenyl group having a carbon number of 2 to 8, an alkynyl group having a carbon number of 2 to 8) and the like.
Examples of the aromatic ring group for $R^1$ include an aryl group having a carbon number of 6 to 14 (e.g., a phenyl group, a naphthyl group etc.).
Examples of the atom and substituent that can substitute the hydrocarbon group and aromatic ring group include those similar to substituents for the aromatic ring group and the heteroaromatic ring group of Ar.
Examples of the metal salt of carboxylic acid include sodium salt, potassium salt, cesium salt, magnesium salt, calcium salt and the like. In addition, examples of the organic salt of the carboxylic acid include ammonium salt and the like.

Specific examples of the carboxylic acid, metal carboxylate and carboxylic acid organic salt include substituted and unsubstituted carboxylic acids such as acetic acid, propionic acid, acrylic acid, phenylacetic acid, benzoic acid, chlorobenzoic acid and the like, metal salts of carboxylic acid such as sodium salt, potassium salt, cesium salt, magnesium salt, calcium salt and the like thereof, and organic salts of carboxylic acid such as ammonium salt, triethylammonium salt and the like thereof.

The amount of carboxylic acid or carboxylic acid salt to be used is preferably 0.5 to 5 equivalents, more preferably 1 to 2 equivalents, relative to optically active 2-aryl-2-halomethyl-4-chloromethyl-1,3-dioxolane (total amount when it is a mixture of a trans form and a cis form, hereinafter the same).

A solvent is generally used in this step. The kind of the solvent is not particularly limited, and aromatic hydrocarbon solvents such as benzene, toluene and xylene, aprotic solvents such as DMF, DMA, DMSO etc., and the like can be used.
The reaction temperature may be about 0˚C to 180˚C. In addition, the reaction time may be generally 1 to 24 hr.

When optically active 2-aryl-2-halomethyl-4-chloromethyl-1,3-dioxolane having a smaller halogen-exchanged compound content, which is obtained by the above-mentioned step (2), is used as a starting material, optically active (2-aryl-2-halomethyl-1,3-dioxolan-4-yl)methyl carboxylates having a smaller content of a halogen-exchanged compound than that of known ones can be obtained.
To be specific, (2S,cis)-(2-aryl-2-halomethyl-1,3-dioxolan-4-yl)methyl carboxylate represented by the formula [VI] and (2R,cis)-(2-aryl-2-halomethyl-1,3-dioxolan-4-yl)methyl carboxylate represented by the formula, which have a content percentage of a halogen-exchanged compound, wherein $X^1$ is exchanged with other halogen group, of not more than

0.2% (preferably not more than 0.1) can be obtained. This compound is novel.
In this case, an embodiment wherein $X^1$ is a halogen atom other than a chlorine atom, which has a content percentage of a halogen-exchanged compound wherein $X^1$ is a chlorine atom of not more than 0.2% (preferably not more than 0.1) is preferable.
Furthermore, an embodiment wherein $X^1$ is a bromine atom, Ar is 4-chlorophenyl, 2,4-dichlorophenyl, 4-fluorophenyl, 2,4-difluorophenyl, 4-chloro-2-trifluoromethylphenyl, phenyl, 4-methylphenyl, 4-biphenyl, 2-naphthyl, 4-methoxyphenyl, 4-phenoxyphenyl, 4-nitrophenyl or 2-thienyl, and $R^1$ is phenyl, methyl, ethyl, 4-nitrophenyl, 4-methoxyphenyl, 4-chlorophenyl or heptyl is preferable.

(4) Synthesis of optically active 2-aryl-2-halomethyl-1,3-dioxolan-4-methanol

The optically active 2-aryl-2-halomethyl-1,3-dioxolan-4-methanol can be synthesized by hydrolyzing optically active (2-aryl-2-halomethyl-1,3-dioxolan-4-yl)methyl carboxylate.
As mentioned above, since 1,3-dioxolane ring is decomposed under acidic conditions, hydrolysis is preferably performed under basic conditions using sodium hydroxide, potassium hydroxide and the like. In addition, the solvent is not particularly limited as long as it is not decomposed under alkaline conditions, and ether solvents such as dioxane, THF and the like, alcohols such as methanol, ethanol and the like, polar solvents such as water, acetone, DMF etc., and the like can be used. The reaction temperature may be about -30˚C to 80˚C, and the reaction time may be generally 1 to 24 hr.

(5) Synthesis of optically active (2-aryl-2-halomethyl-1,3-dioxolan-4-yl)methyl sulfonate

The optically active (2-aryl-2-halomethyl-1,3-dioxolan-4-yl)methyl sulfonate can be synthesized by reacting the optically active 2-aryl-2-halomethyl-1,3-dioxolan-4-methanol with sulfonyl halide, sulfonic anhydride or the like in a suitable solvent in the presence of a base.
The sulfonyl halide and sulfonic anhydride to be used in this step pose no particular problem as long as they can be generally used for the synthesis of sulfonate from alcohol. For example, sulfonyl halide represented by the formula: $R^2SO_2X^3$ and sulfonic anhydride represented by the formula: $(R^2SO_2)_2O$, wherein $R^2$ is a substituted or unsubstituted hydrocarbon group, or a substituted or unsubstituted aromatic ring group, and $X^3$ is a halogen atom, can be mentioned.
The "substituted or unsubstituted hydrocarbon group" and "substituted or unsubstituted aromatic ring group" for $R^2$ are similar to the above-mentioned "substituted or unsubstituted hydrocarbon group" and "substituted or unsubstituted aromatic ring group" for $R^1$.
Examples of the halogen atom for $X^3$ include a chlorine atom and a bromine atom.
As generally available sulfonyl halide and sulfonic anhydride, methanesulfonyl chloride, toluenesulfonyl chloride, nitrobenzenesulfonyl chloride, trifluorobenzenesulfonic anhydride and the like can be mentioned.
The amount of sulfonyl halide or sulfonic anhydride to be used is preferably 0.5 to 5 equivalents, more preferably 0.5 to 2 equivalents, relative to optically active 2-aryl-2-halomethyl-1,3-dioxolan-4-methanol.
Examples of the base to be used include organic bases such as triethylamine, pyridine and the like, and inorganic bases such as sodium carbonate, potassium carbonate, sodium hydrogen carbonate, sodium hydroxide and the like.
The amount of the base to be used is preferably 0.5 to 5 equivalents, more preferably 1 to 2 equivalents, relative to optically active 2-aryl-2-halomethyl-1,3-dioxolan-4-methanol.
The solvent to be used is not particularly limited as long as it does not react with a substrate, and ether solvents such as dioxane, THF and the like, ester solvents such as ethyl acetate and the like, chlorine solvents such as methylene chloride etc., and the like can be mentioned.
The reaction temperature may be about -30˚C to 50˚C, and the reaction time may be generally 1 to 48 hr.
Used for the induction to ketoconazole are optically active cis-(2-aryl-2-halomethyl-1,3-dioxolan-4-yl)methyl carboxylate and optically active cis-(2-aryl-2-halamethyl-1,3-dioxolan-4-yl)methyl sulfonate, and when they are obtained as mixtures of a cis form and a trans form, the cis form can be isolated from the mixtures by a known isolation and purification means (crystallization, recrystallization, column chromatography etc.). Preferably, the isolation can be easily performed by recrystallization.

(6) Isolation of optically active cis-(2-aryl-2-halomethyl-1,3-dioxolan-4-yl)methyl sulfonate

While the method of isolating the optically active cis-(2-aryl-2-halomethyl-1,3-dioxolan-4-yl)methyl sulfonate from a mixture containing optically active trans-(2-aryl-2-halomethyl-1,3-dioxolan-4-yl)methyl sulfonate and optically active cis-(2-aryl-2-halomethyl-1,3-dioxolan-4-yl)methyl sulfonate is not particularly limited as long as it can isolate optically active cis-(2-aryl-2-halomethyl-1,3-dioxolan-4-yl)methyl sulfonate, isolation can be performed by recrystallization using a suitable solvent, by utilizing the tendency toward preferential crystallization of a cis form to a trans form.

As the solvent, alcohols such as methanol, ethanol, isopropanol and the like, hydrocarbon solvents such as toluene, hexane and the like, ether solvents such as THF and the like, ester solvents such as ethyl acetate and the like, chlorine solvents such as methylene chloride and the like, polar solvents such as water, acetone, DMF etc., and the like can be used. In addition, two or more kinds of solvents may be used in combination. Preferred are alcohol solvents such as methanol and the like, and water.

Examples of the recrystallization method include a method comprising dissolving a mixture containing optically active trans-(2-aryl-2-halomethyl-1,3-dioxolan-4-yl)methyl sulfonate and optically active cis-(2-aryl-2-halomethyl-1,3-dioxolan-4-yl)methyl sulfonate in a solvent with heating, and then cooling, a method comprising dissolving the mixture in a solvent, and then concentrating the solution, a method comprising dissolving the mixture in a solvent, and then adding a poor solvent (hydrocarbon solvents such as hexane etc., water and the like), a combination of these methods and the like.

The temperature of crystallization may be about -30˚C to 80˚C, and the crystallization time may be generally 1 to 24 hr.

(7) Isolation of optically active cis-(2-aryl-2-halomethyl-1,3-dioxolan-4-yl)methyl carboxylate

While a method for isolating optically active cis-(2-aryl-2-halomethyl-1,3-dioxolan-4-yl)methyl carboxylate from a mixture containing optically active trans-(2-aryl-2-halomethyl-1,3-dioxolan-4-yl)methyl carboxylate and optically active cis-(2-aryl-2-halomethyl-1,3-dioxolan-4-yl)methyl carboxylate is not particularly limited as long as it can isolate optically active cis-(2-aryl-2-halomethyl-1,3-dioxolan-4-yl)methyl carboxylate, isolation can be performed by recrystallization using a suitable solvent, by utilizing the tendency toward preferential crystallization of a cis form to a trans form.

As the solvent, alcohols such as methanol, ethanol, isopropanol and the like, hydrocarbon solvents such as toluene, hexane and the like, ether solvents such as THF and the like, ester solvents such as ethyl acetate and the like, chlorine solvents such as methylene chloride and the like, polar solvents such as water, acetone, DMF etc., and the like can be used. In addition, two or more kinds of solvents may be used in combination. Preferably are alcohol solvents such as methanol and the like, and water.

Examples of the recrystallization method include a method comprising dissolving a mixture containing optically active trans-(2-aryl-2-halomethyl-1,3-dioxolan-4-yl)methyl carboxylate and optically active cis-(2-aryl-2-halomethyl-1,3-dioxolan-4-yl)methyl carboxylate in a solvent with heating, and then cooling the solution, a method comprising dissolving the mixture in a solvent, and then concentrating the solution, a method comprising dissolving the mixture in a solvent, and then adding a poor solvent (hydrocarbon solvents such as hexane etc., water and the like), or a combination of these methods and the like.

The temperature of crystallization may be about -30˚C to 80˚C, and the crystallization time may be generally 1 to 24 hr.

The mother liquid of the recrystallization step contains a mixture of optically active trans-(2-aryl-2-halomethyl-1,3-dioxolan-4-yl)methyl carboxylate and optically active cis-(2-aryl-2-halomethyl-1,3-dioxolan-4-yl)methyl carboxylate in an amount smaller than that of the former. By isomerizing the optically active trans-(2-aryl-2-halomethyl-1,3-dioxolan-4-yl)methyl carboxylate contained in the mixture to a cis form, a mixture containing the cis form in an amount equivalent to or higher than that of the trans form can be induced.

In addition, in the mother liquid or a mixture containing optically active trans-(2-aryl-2-halomethyl-1,3-dioxolan-4-yl) methyl carboxylate and optically active cis-(2-aryl-2-halomethyl-1,3-dioxolan-4-yl)methyl carboxylate, which is obtained by the above-mentioned step (3), the yield of the cis form can be further improved by crystallizing the cis form while isomerizing the trans form to a cis form.

The isomerization is explained in detail in the following.

(8) Isomerization of optically active trans-(2-aryl-2-halomethyl-1,3-dioxolan-4-yl)methyl carboxylate to optically active cis-(2-aryl-2-halomethyl-1,3-dioxolan-4-yl)methyl carboxylate

This step includes at least a step of isomerizing optically active trans-(2-aryl-2-halomethyl-1,3-dioxolan-4-yl) methyl carboxylate (isomerization step).

The optically active trans-(2-aryl-2-halomethyl-1,3-dioxolan-4-yl)methyl carboxylate is isomerized to optically active cis-(2-aryl-2-halomethyl-1,3-dioxolan-4-yl)methyl carboxylate as shown below.

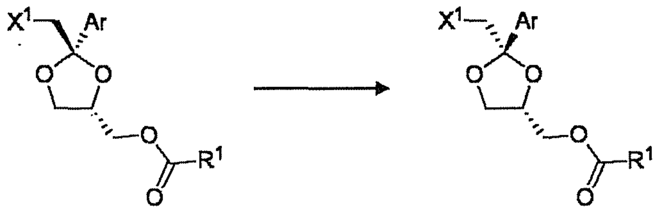

[V] [VI]

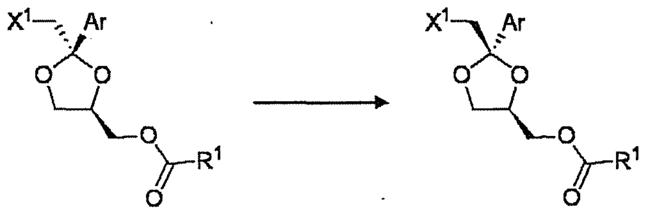

[XIV] [XV]

wherein each symbol is as defined above.

The optically active trans-(2-aryl-2-halomethyl-1,3-dioxolan-4-yl)methyl carboxylate of the formula [V] or the formula [XIV] to be used in the isomerization step of the present invention is not limited to pure ones scarcely containing a cis form, and may be a mixture of a trans form and a cis form.

As the acid catalyst to be used in the isomerization step of the present invention, Lewis acid, organic acid, inorganic acid and the like can be used. Examples of the Lewis acid include boron trifluoride-diethyl ether complex ($BF_3 \cdot Et_2O$), ferric chloride, aluminum chloride, tin chloride and the like. Examples of the organic acid include methanesulfonic acid, toluenesulfonic acid, acetic acid, trifluoroacetic acid and the like, and examples of the inorganic acid include hydrogen chloride, hydrobromic acid, sulfuric acid and the like. Of these, a Lewis acid or an organic acid is preferably used, since decomposition of substrate and the like are suppressed, and a high yield can be achieved.

The amount of the acid catalyst to be used is appropriately 0.1 to 50 equivalents, more preferably 0.5 to 30 equivalents, relative to optically active (2-aryl-2-halomethyl-1,3-dioxolan-4-yl)methyl carboxylate (total amount when it is a mixture of a trans form and a cis form, hereinafter the same). When the amount of the acid catalyst to be used is less than 0.1 equivalent relative to optically active (2-aryl-2-halomethyl-1,3-dioxolan-4-yl)methyl carboxylate, isomerization unpreferably requires a long time. On the other hand, when it exceeds 50 equivalents relative to optically active (2-aryl-2-halomethyl-1,3-dioxolan-4-yl)methyl carboxylate, it is unpreferably uneconomical.

While the solvent to be used for the isomerization step of the present invention is not particularly limited, aliphatic hydrocarbon solvents such as hexane, heptane, cyclohexane and the like, aromatic hydrocarbon solvents such as benzene, toluene, xylene and the like, halogenated hydrocarbon solvents such as dichloromethane, chloroform, carbon tetrachloride, 1,2-dichloroethane and the like, nitrile solvents such as acetonitrile, propionitrile and the like, ester solvents such as methyl acetate, ethyl acetate and the like are preferable, since the isomerization proceeds efficiently. In addition, these solvents can also be used in a mixture at a desired ratio. Furthermore, an acid catalyst may be used singly instead of the solvent, without using a solvent.

While the amount of the solvent to be used is not particularly limited, it is generally 0 to 20 parts by weight, preferably 1 to 10 parts by weight, relative to 1 part by weight of optically active (2-aryl-2-halomethyl-1,3-dioxolan-4-yl) methyl carboxylate.

While the reaction temperature is not particularly limited in the isomerization step of the present invention, it is preferably 0 to 60°C. When the temperature is too low, the reaction time tends to be long. In addition, the reaction may be performed under normal pressure or can be performed under reduced pressure. The reaction time may be generally 0.5 to 48 hr.

In the isomerization step of the present invention, a cis form and a trans form in the isomerization treatment liquid is in equilibrium, and the ratio of the cis form and the trans form in the isomerization treatment liquid after reaching the equilibrium is about 40:60 to 60:40. However, it is also possible to obtain optically active cis-(2-aryl-2-halomethyl-1,3-dioxolan-4-yl)methyl carboxylate in a larger amount as a whole reaction system, by performing isomerization while allowing precipitation of optically active cis-(2-aryl-2-halomethyl-1,3-dioxolan-4-yl)methyl carboxylate as crystals in the isomerization step. To perform isomerization while allowing precipitation of optically active cis-(2-aryl-2-halomethyl-1,3-dioxolan-4-yl)methyl carboxylate as crystals, the amount of the solvent to be used relative to the substrate may be

reduced, the reaction may be performed while evaporating the solvent, a poor solvent may be added, or the reaction mixture may be cooled, and the like. In this case, the ratio of the cis form and the trans form as a whole reaction system can be increased to about 60:40 to 95:5.

Since the optically active cis-(2-aryl-2-halomethyl-1,3-dioxolan-4-yl)methyl carboxylate obtained by the isomerization step of the present invention is obtained as a mixture with optically active trans-(2-aryl-2-halomethyl-1,3-dioxolan-4-yl)methyl carboxylate, a step of isolating optically active cis-(2-aryl-2-halomethyl-1,3-dioxolan-4-yl)methyl carboxylate (isolation step) is preferably provided. The isolation step may be performed after other step(s) following the isomerization step, such as neutralization, purification and the like.

The optically active cis-(2-aryl-2-halomethyl-1,3-dioxolan-4-yl)methyl carboxylate can be isolated by generally-performed operations, such as recrystallization, column chromatography and the like. Of these, recrystallization is desirable in consideration of the industrial treatment of large amounts. One example of the recrystallization operation is given. A base is added to an isomerization treatment liquid to quench an acid catalyst to cease the equilibrium reaction, and the organic layer is concentrated to dryness by a rotary evaporator and the like. Then, the residue obtained by the concentration to dryness is recrystallized from a suitable poor solvent such as methanol and the like to give optically active cis-(2-aryl-2-halomethyl-1,3-dioxolan-4-yl)methyl carboxylate as crystals. The recrystallization can be preferably performed in the same manner as in the above-mentioned step (3).
Examples of the base for discontinuing the equilibrium reaction include organic bases such as triethylamine, pyridine and the like, inorganic bases such as sodium carbonate, potassium carbonate etc., and the like.

In the isolation step, isolation of optically active cis-(2-aryl-2-halomethyl-1,3-dioxolan-4-yl)methyl carboxylate may be performed by not only subjecting a mixture of a cis form and a trans form of the optically active (2-aryl-2-halomethyl-1,3-dioxolan-4-yl)methyl carboxylate obtained by the isomerization step of the present invention to the isolation step, but also simultaneously subjecting a mixture of a cis form and a trans form of optically active (2-aryl-2-halomethyl-1,3-dioxolan-4-yl)methyl carboxylate obtained by other synthesis pathway, and a mixture of a cis form and a trans form obtained by the isomerization step of the present invention to the isolation step to isolate optically active cis-(2-aryl-2-halomethyl-1,3-dioxolan-4-yl)methyl carboxylate.

Furthermore, the optically active cis-(2-aryl-2-halomethyl-1,3-dioxolan-4-yl)methyl carboxylate isolated in step (7) or (8) may be used as a starting material and led to optically active cis-(2-aryl-2-halomethyl-1,3-dioxolan-4-yl)methyl sulfonate according to steps (4) and (5).

The optically active (2-aryl-2-halomethyl-1,3-dioxolan-4-yl)methyl carboxylate and optically active (2-aryl-2-halomethyl-1,3-dioxolan-4-yl)methyl sulfonate obtained by the above-mentioned steps can be led to ketoconazole according to, for example, the description of Bauer, L. et al., J. Heterocycl. Chem. 1990, 27, p.2053-2061.

## Examples

The present invention is explained in the following by referring to Examples, which are not to be construed as limitative. When the compound names below include trans-,cis-, it means the compound is a mixture of a trans form and a cis form.

### Example 1

Synthesis of (4S)-trans-,cis-2-(4-chlorophenyl)-2-bromomethyl-4-chloromethyl-1,3-dioxolane

A mixture of 2-bromo-4'-chloroacetophenone (4.94 g, 2-chloro-4'-chloroacetophenone content = 0.09%), (S)-monochlorohydrin (2.59 g, 1.1 equivalents, >99%ee), p-toluenesulfonic acid monohydrate (0.20 g, 0.05 equivalent) and toluene (100 mL) was refluxed at 130˚C using an azeotropic distillation device with a Dean-Stark tube. After confirmation of the completion of the azeotropic distillation, the reaction mixture was cooled and washed with 10% aqueous sodium hydrogen carbonate solution and 10% brine. The solvent was evaporated under reduced pressure to give (4S)-trans-,cis-2-(4-chlorophenyl)-2-bromomethyl-4-chloromethyl-1,3-dioxolane (6.36 g, >99%ee, trans/cis=34/66) (desired product). Here, the content percentage of halogen-exchanged (4S)-trans-,cis-2-(4-chlorophenyl)-2-chloromethyl-4-chloromethyl-1,3-dioxolane (halogen-exchanged compound) was 1.3%. The halogen-exchanged compound content percentage was calculated as follows. halogen-exchanged compound content percentage

(%)=100×(halogen-exchanged compound amount)/{(desired product amount)+(halogen-exchanged compound amount)}

Examples 2 to 16

Synthesis of (4S)-trans-,cis-2-aryl-2-halomethyl-4-chloromethyl-1,3-dioxolane

The reactions of Examples 2 to 16 were performed according to Example 1 and using aryl(halomethyl)ketones shown in Table 1 and Table 2. The results are shown in Table 3 and Table 4 together with Example 1.

Table 1

| Example | aryl(halomethyl)ketone | halogen-exchanged compound | halogen-exchanged compound content (%) |
|---|---|---|---|
| 1 | 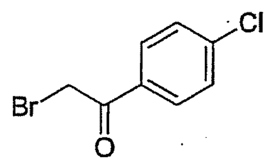 | 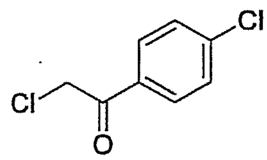 | 0.09 |
| 2 | 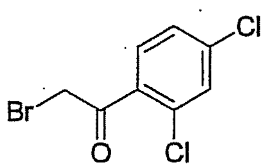 | 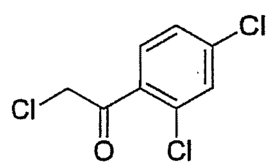 | 0.08 |
| 3 | 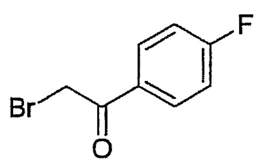 | 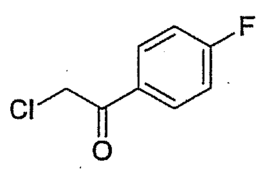 | 0.09 |
| 4 | 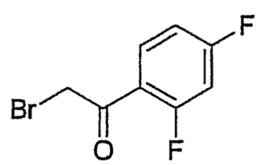 | 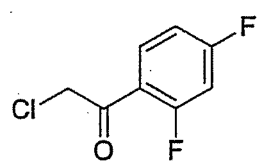 | 0.07 |
| 5 | 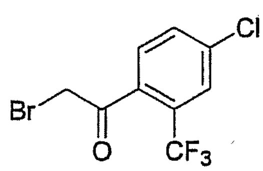 | 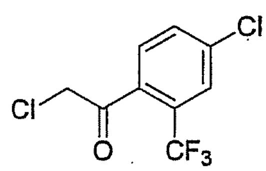 | 0.09 |
| 6 | 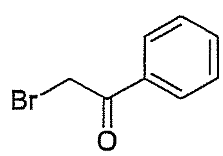 | 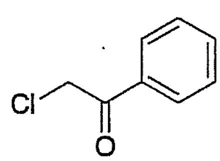 | 0.06 |
| 7 | 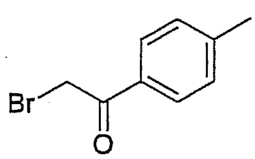 | 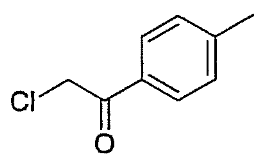 | 0.07 |

(continued)

| Example | aryl(halomethyl)ketone | halogen-exchanged compound | halogen-exchanged compound content (%) |
|---|---|---|---|
| 8 | 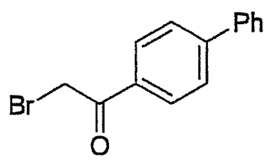 | 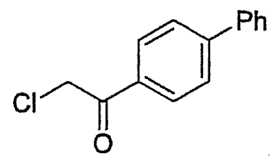 | 0.08 |
| 9 | 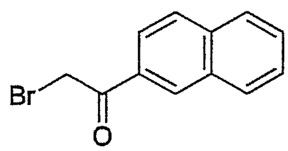 | 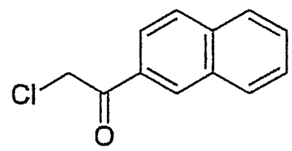 | 0.08 |

Table 2

| Example | aryl(halomethyl)ketone | halogen-exchanged compound | halogen-exchanged compound content (%) |
|---|---|---|---|
| 10 | 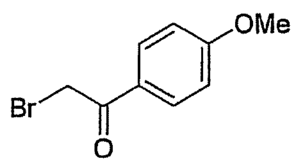 | 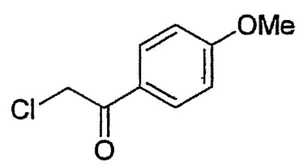 | 0.07 |
| 11 | 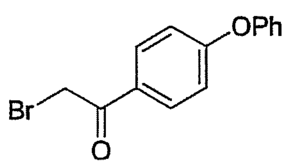 | 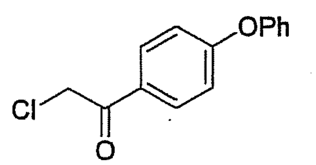 | 0.09 |
| 12 | 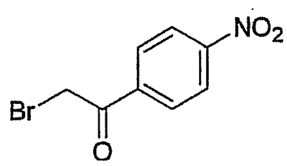 | 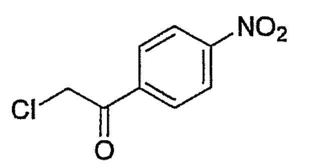 | 0.08 |
| 13 | 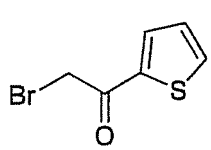 | 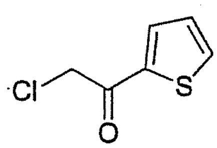 | 0.09 |
| 14 | 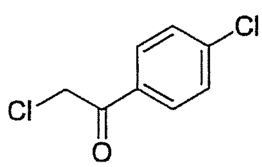 | - | 0 |
| 15 | 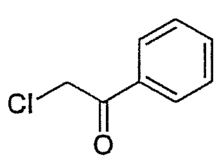 | - | 0 |

(continued)

| Example | aryl(halomethyl)ketone | halogen-exchanged compound | halogen-exchanged compound content (%) |
|---|---|---|---|
| 16 | 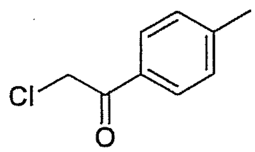 | - | 0 |

Table 3

| Example | desired product | | halogen-exchanged compound | yield (%) of desired product | cis/trans | halogen-exchanged compound content (%) |
|---|---|---|---|---|---|---|
| | cis | trans | | | | |
| 1 | 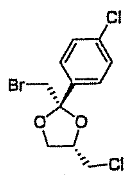 | 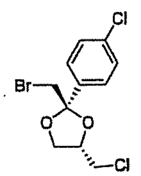 | 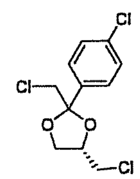 | 92 | 66/34 | 1. 3 |
| 2 | 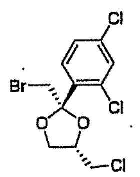 | 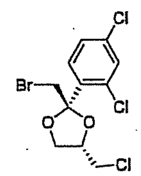 | 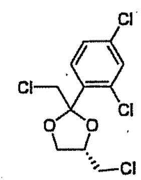 | 91 | 66/34 | 1. 1 |
| 3 | 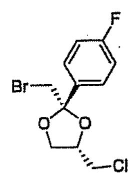 | 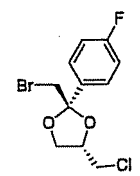 | 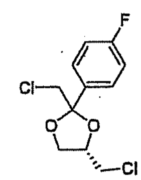 | 92 | 66/34 | 1. 2 |
| 4 | 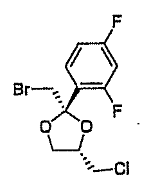 | 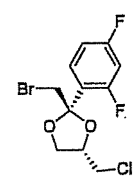 | 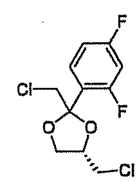 | 89 | 65/35 | 1. 2 |
| 5 | 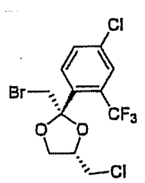 | 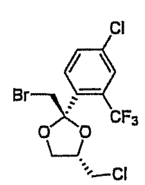 | 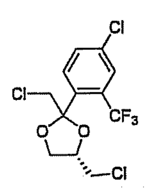 | 93 | 66/34 | 1. 1 |
| 6 | 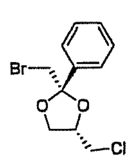 | 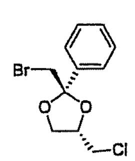 | 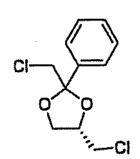 | 92 | 66/34 | 1. 3 |

(continued)

| Example | desired product | | halogen-exchanged compound | yield (%) of desired product | cis/trans | halogen-exchanged compound content (%) |
|---|---|---|---|---|---|---|
| | cis | trans | | | | |
| 7 | 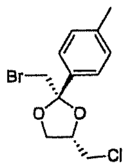 | 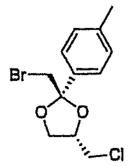 | 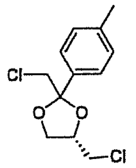 | 90 | 67/33 | 1. 3 |
| 8 | 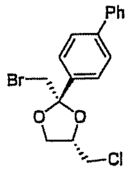 | 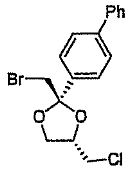 | 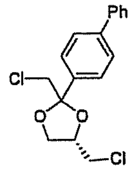 | 89 | 66/34 | 1. 2 |
| 9 | 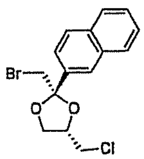 | 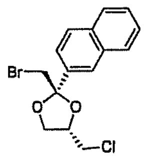 | 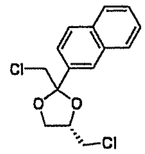 | 87 | 66/34 | 1. 2 |

[Table 4]

| Example | desired product | | halogen-exchanged compound | yield (%) of desired product | cis/trans | halogen-exchanged compound content (%) |
|---|---|---|---|---|---|---|
| | cis | trans | | | | |
| 10 | 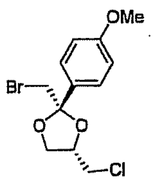 | 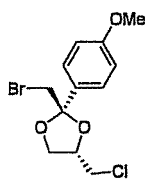 | 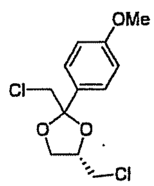 | 90 | 66/34 | 1. 3 |
| 11 | 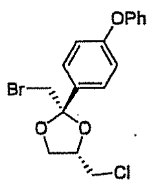 | 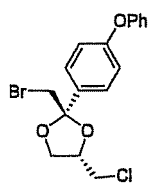 | 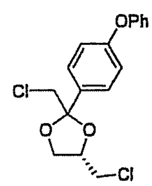 | 94 | 66/34 | 1. 2 |
| 12 | 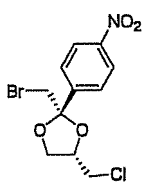 | 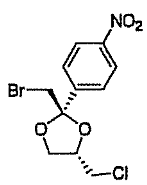 | 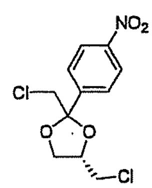 | 89 | 66/34 | 1. 3 |

(continued)

| Example | desired product | | halogen-exchanged compound | yield (%) of desired product | cis/trans | halogen-exchanged compound content (%) |
|---|---|---|---|---|---|---|
| | cis | trans | | | | |
| 13 | 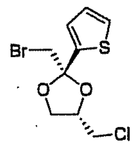 | 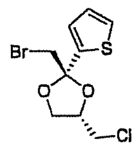 | 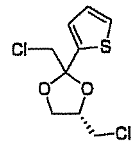 | 88 | 66/34 | 1. 2 |
| 14 | 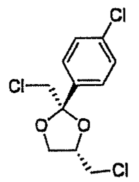 | 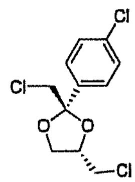 | - | 98 | 67/33 | 0 |
| 15 | 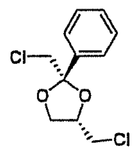 | 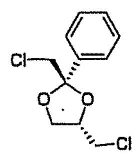 | - | 98 | 66/34 | 0 |
| 16 | 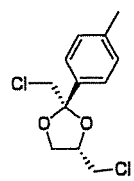 | 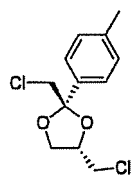 | - | 97 | 66/34 | 0 |

Examples 17 to 22

Synthesis of (4S)-trans-,cis-2-(4-chlorophenyl)-2-bromomethyl-4-chloromethyl-1,3-dioxolane using various acid catalysts

In Examples 17 to 22, the reactions were performed by changing only the acid catalyst in the reaction of Example 1 to various acid catalysts (0.05 equivalent). The results are shown in Table 5 together with Example 1.

[Table 5]

| Example | acid catalyst | yield (%) of desired product | cis/trans | halogen-exchanged compound content (%) |
|---|---|---|---|---|
| 1 | $TsOH{\cdot}H_2O$ | 92 | 66/34 | 1. 3 |
| 17 | MsOH | 89 | 66/34 | 1. 3 |
| 18 | $CH_3COOH$ | 87 | 66/34 | 1. 3 |
| 19 | $BF_3{\cdot}OEt_2$ | 90 | 65/35 | 1. 3 |
| 20 | $H_2SO_4$ | 88 | 66/34 | 1. 3 |
| 21 | HCl | 88 | 66/34 | 1. 8 |
| 22 | HBr | 93 | 66/34 | 1. 1 |

Example 23

Synthesis of (4S)-trans-,cis-2-(4-chlorophenyl)-2-bromomethyl-4-chloromethyl-1,3-dioxolane suppressing halogen exchange between substrates

A mixture of 2-bromo-4'-chloroacetophenone (4.94 g, 2-chloro-4'-chloroacetophenone content=0.09%), p-toluenesulfonic acid monohydrate (0.20 g, 0.05 equivalent) and toluene (100 mL) was refluxed at 130°C using an azeotropic distillation device with a Dean-Stark tube, and (S)-monochlorohydrin (2.59 g, 1.1 equivalents, >99%ee) was added dropwise under reflux such that the amount of the (S)-monochlorohydrin present in the reaction solution would be not more than 0.1 equivalent (not more than 2.1 mmol) relative to the amount of 2-bromo-4'-chloroacetophenone to be used (21.2 mmol), while analyzing the progress of the reaction by GC.
After confirmation of the completion of the azeotropic distillation, the reaction mixture was cooled and washed with 10% aqueous sodium hydrogen carbonate solution and 10% brine. The solvent was evaporated under reduced pressure to give (4S)-trans-,cis-2-(4-chlorophenyl)-2-bromomethyl-4-chloromethyl-1,3-dioxolane (6.56 g, >99%ee). Here, the content percentage of (4S)-trans-,cis-2-(4-chlorophenyl)-2-chloromethyl-4-chloromethyl-1,3-dioxolane halogen-exchanged with a chlorine atom was 0.09%.

Examples 24 to 29

Synthesis of (4S)-trans-,cis-2-(4-chlorophenyl)-2-bromomethyl-4-chloromethyl-1,3-dioxolane suppressing halogen exchange due to acid catalyst

In Examples 24 to 29, the reactions were performed by changing only the acid catalyst in the reaction of Example 23 to various acid catalysts (0.05 equivalent). The results are shown in Table 6 together with Example 23.
[Table 6]

| Example | acid catalyst | yield (%) of desired product | cis/trans | halogen-exchanged compound content (%) |
|---|---|---|---|---|
| 23 | $TsOH \cdot H_2O$ | 94 | 66/34 | 0. 09 |
| 24 | MsOH | 91 | 66/34 | 0. 09 |
| 25 | $CH_3COOH$ | 88 | 66/34 | 0. 09 |
| 26 | $BF_3 \cdot OEt_2$ | 93 | 66/34 | 0. 09 |
| 27 | $H_2SO_4$ | 87 | 66/34 | 0. 09 |
| 28 | HCl | 94 | 66/34 | 1. 2 |
| 29 | HBr | 93 | 66/34 | 0. 09 |

Examples 30 to 41

Synthesis of (4S)-trans-cis-2-aryl-2-bromomethyl-4-chloromethyl-1,3-dioxolane suppressing halogen exchange

In Examples 30 to 41, reactions were performed according to Example 23 and using aryl(bromomethyl)ketones (halogen-exchanged compound content<0.1%) shown in Table 7 and Table 8. The results are shown in Table 9 and Table 10 together with Example 23.
[Table 7]

| Example | aryl(halomethyl)ketone | halogen-exchanged compound | halogen-exchanged compound content (%) |
|---|---|---|---|
| 23 | 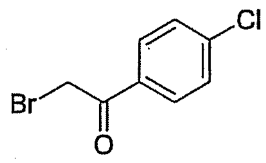 | 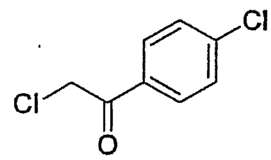 | 0.09 |
| 30 | 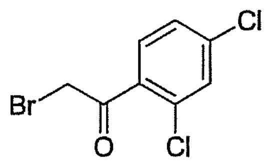 | 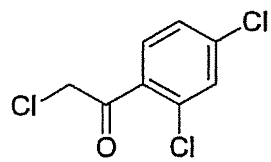 | 0.08 |
| 31 | 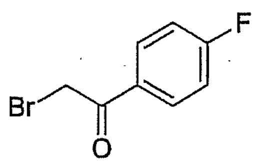 | 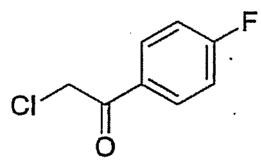 | 0.09 |
| 32 | 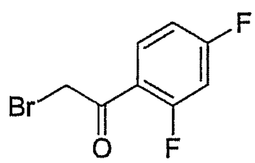 | 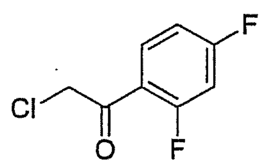 | 0.07 |
| 33 | 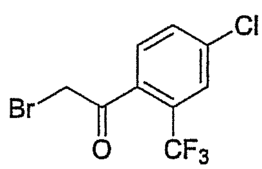 | 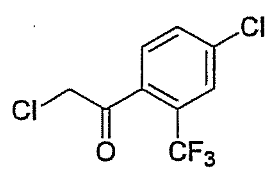 | 0.09 |
| 34 | 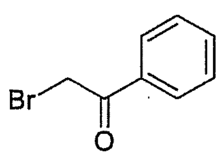 | 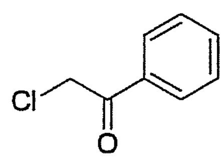 | 0.06 |
| 35 | 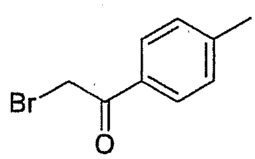 | 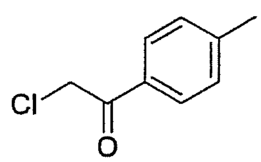 | 0.07 |
| 36 | 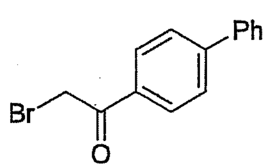 | 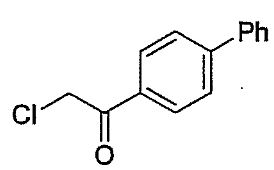 | 0.08 |
| 37 | 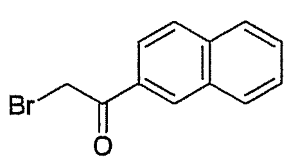 | 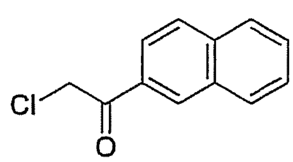 | 0.08 |

[Table 8]

| Example | aryl(halomethyl)-ketone | halogen-exchanged compound | halogen-exchanged compound content (%) |
|---|---|---|---|
| 38 | 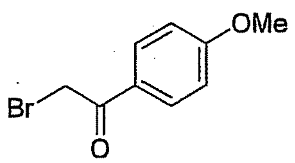 | 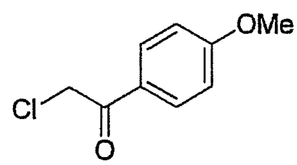 | 0.07 |
| 39 | 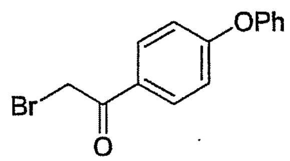 | 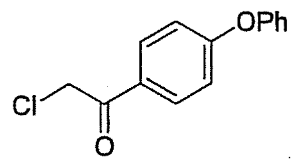 | 0.09 |
| 40 | 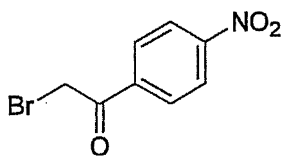 | 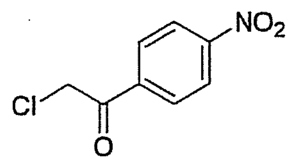 | 0.08 |
| 41 | 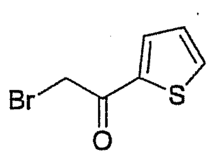 | 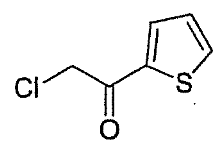 | 0.09 |

[Table 9]

| Example | desired product | | halogen-exchanged compound | yield (%) of desired product | cis/trans | halogen-exchanged compound content |
|---|---|---|---|---|---|---|
| | cis | trans | | | | |
| 23 | 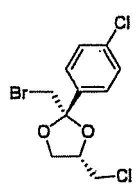 | 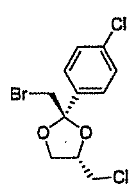 | 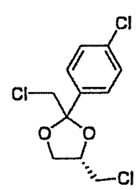 | 94 | 66/34 | 0.09 |
| 30 | 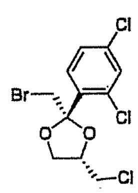 | 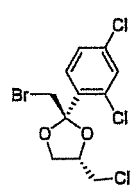 | 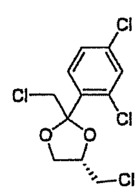 | 92 | 66/34 | 0.08 |
| 31 | 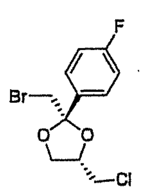 | 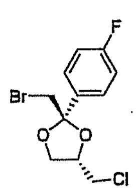 | 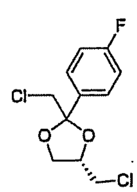 | 93 | 66/34 | 0.09 |

(continued)

| Example | desired product | | halogen-exchanged compound | yield (%) of desired product | cis/trans | halogen-exchanged compound content |
|---|---|---|---|---|---|---|
| | cis | trans | | | | |
| 32 | 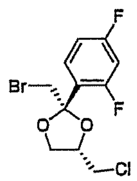 | 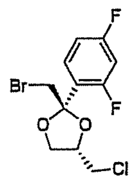 | 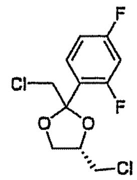 | 92 | 67/33 | 0.07 |
| 33 | 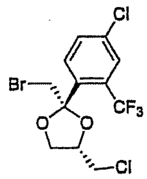 | 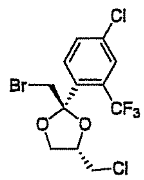 | 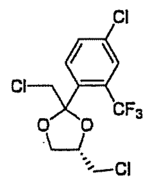 | 94 | 66/34 | 0.09 |
| 34 | 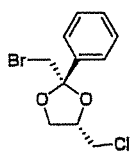 | 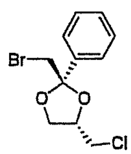 | 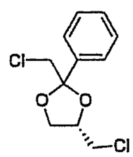 | 94 | 66/34 | 0.06 |
| 35 | 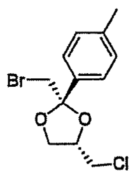 | 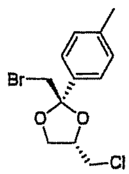 | 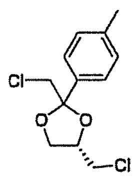 | 94 | 66/34 | 0.07 |
| 36 | 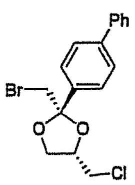 | 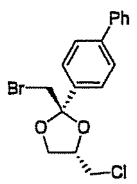 | 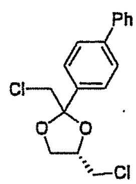 | 91 | 65/35 | 0.08 |
| 37 | 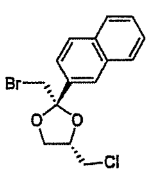 | 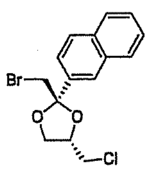 | 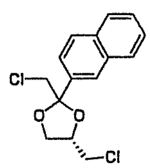 | 92 | 66/34 | 0.08 |

[Table 10]

| Example | desired product | | halogen-exchanged compound | yield (%) of desired product | cis/trans | halogen-exchanged compound content (%) |
|---|---|---|---|---|---|---|
| | cis | trans | | | | |
| 38 | 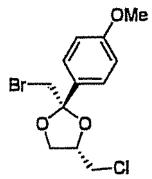 | 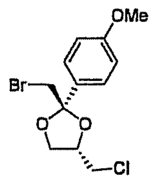 | 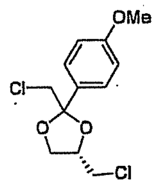 | 90 | 67/33 | 0.07 |
| 39 | 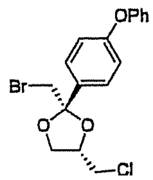 | 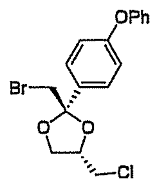 | 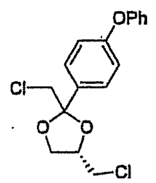 | 93 | 66/34 | 0.09 |
| 40 | 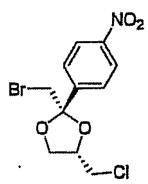 | 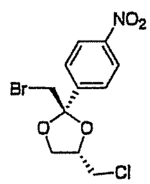 | 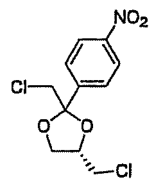 | 91 | 66/34 | 0.08 |
| 41 | 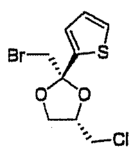 | 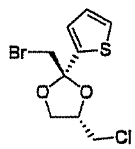 | 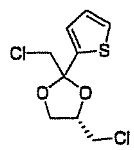 | 89 | 65/35 | 0.09 |

Example 42

Synthesis of (4S)-trans-,cis-[2-(4-chlorophenyl)-2-bromomethyl-1,3-dioxolan-4-yl]methyl benzoate

A mixture of (4S)-trans-,cis-2-(4-chlorophenyl)-2-bromomethyl-4-chloromethyl-1,3-dioxolane (6.36 g) synthesized in Example 1, sodium benzoate (2.88 g, 1.0 equivalent) and DMF (100 mL) was stirred with heating at 150˚C for 8 hr. After cooling, the reaction mixture was diluted with water (100 mL), and extracted 3 times with ethyl acetate (100 mL). The organic layer was washed with water, and the solvent was evaporated under reduced pressure to give (4S)-trans-,cis-[2-(4-chlorophenyl)-2-bromomethyl-1,3-dioxolan-4-yl]methyl benzoate (5.46 g, >99%ee, trans/cis=34/66). Here, the content percentage of (4S)-trans-,cis-[2-(4-chlorophenyl)-2-chloromethyl-1,3-dioxolan-4-yl] methyl benzoate, which was derived from the halogen-exchanged compound in Example 1, was also 1.3%.

Examples 43 to 48

Synthesis of (4S)-trans-,cis-[2-(4-chlorophenyl)-2-bromomethyl-1,3-dioxolan-4-yl]methyl carboxylate

In Examples 43 to 48, reactions were performed according to Example 42 and using various carboxylic acid salts. The results are shown in Table 11 together with Example 42.

[Table 11]

| Example | carboxylic acid salt | resulting product | | yield (%) | cis/trans |
|---|---|---|---|---|---|
| | | cis | trans | | |
| 42 | 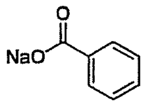 | 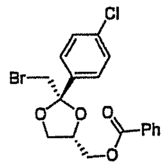 | 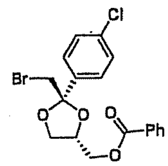 | 68 | 66/34 |
| 43 | 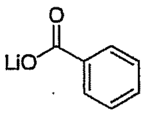 | 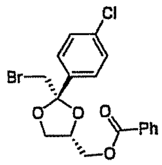 | 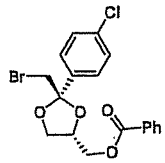 | 63 | 66/34 |
| 44 | 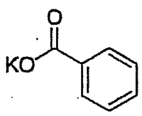 | 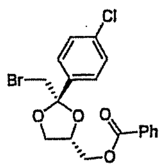 | 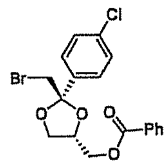 | 61 | 66/34 |
| 45 | 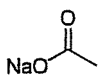 | 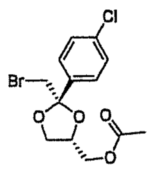 | 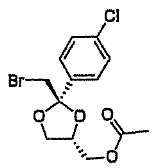 | 57 | 66/34 |
| 46 | 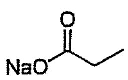 | 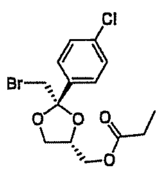 | 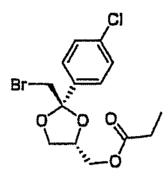 | 59 | 66/34 |
| 47 | 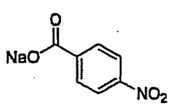 | 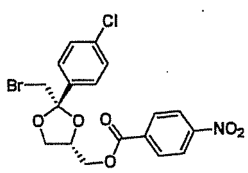 | 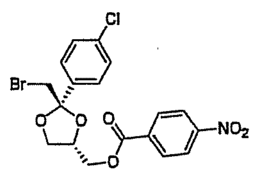 | 59 | 66/34 |
| 48 | 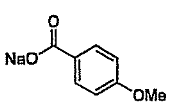 | 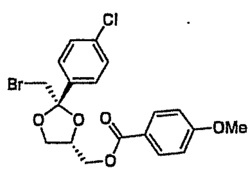 | 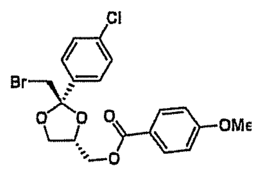 | 63 | 66/34 |

Examples 49 to 51

Synthesis of (4S)-trans-,cis-[2-aryl-2-bromomethyl-1,3-dioxolan-4-yl]methyl benzoate

In Examples 49 to 51, reactions were performed according to Example 42 and using (4S)-trans-,cis-2-aryl-2-halomethyl-4-chloromethyl-1,3-dioxolane in Table 12, which were synthesized in Examples 23, 6 and 34. The results are shown in Table 13.

[Table 12]

| Example | substrate | | | cis/trans | halogen-exchanged compound content (%) |
|---|---|---|---|---|---|
| | cis | trans | halogen-exchanged compound | | |
| 42 | 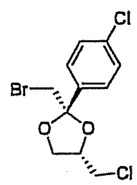 | 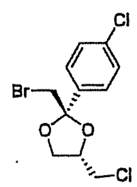 | 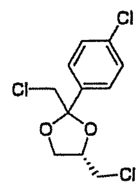 | 66/34 | 1. 3 |
| 49 | 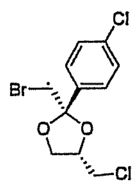 | 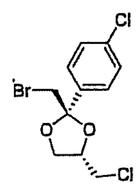 | 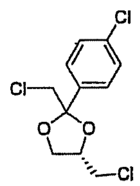 | 66/34 | 0.09 |
| 50 | 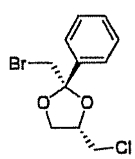 | 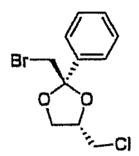 | 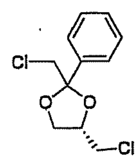 | 66/34 | 1.3 |
| 51 | 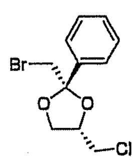 | 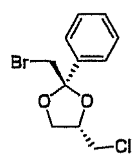 | 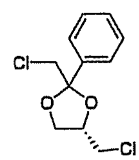 | 66/34 | 0.06 |

[Table 13]

| Example | desired product | | halogen-exchanged compound | yield (%) of desired product | cis/trans | halogen-exchanged compound content (%) |
|---|---|---|---|---|---|---|
| | cis | trans | | | | |
| 42 | 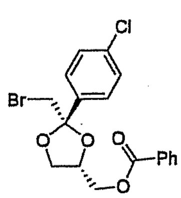 | 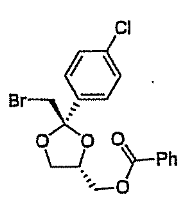 | 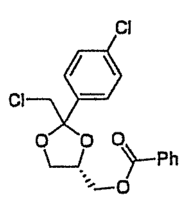 | 68 | 66/34 | 1.3 |

(continued)

| Example | desired product | | halogen-exchanged compound | yield (%) of desired product | cis/trans | halogen-exchanged compound content (%) |
|---|---|---|---|---|---|---|
| | cis | trans | | | | |
| 49 | 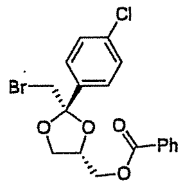 | 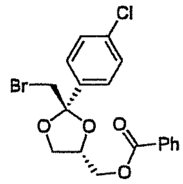 | 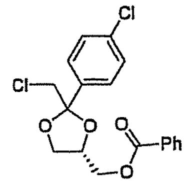 | 70 | 66/34 | 0.09 |
| 50 | 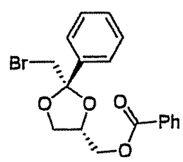 | 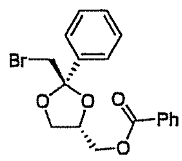 | 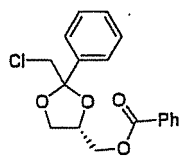 | 69 | 66/34 | 1. 3 |
| 51 | 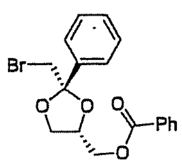 | 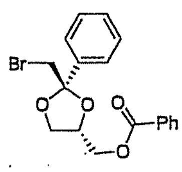 | 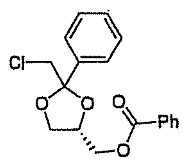 | 70 | 66/34 | 0.06 |

Example 52

Synthesis of (4R)-trans-,cis-2-(4-chlorophenyl)-2-bromomethyl-1,3-dioxolan-4-methanol

To a mixture of (4S)-trans-,cis-[2-(4-chlorophenyl)-2-bromomethyl-1,3-dioxolan-4-yl]methyl benzoate (2.73 g) synthesized in Example 42 and dioxane (67 mL) was added 10% NaOH (4.00 g, 1.5 equivalents) at 5˚C, and the mixture was stirred for 5 hr. The reaction mixture was diluted with water (67 mL), and extracted 3 times with ethyl acetate (67 mL). The organic layer was washed with water, and the solvent was evaporated under reduced pressure to give (4R)-trans-,cis-2-(4-chlorophenyl)-2-bromomethyl-1,3-dioxolan-4-methanol (2.04 g, >99%ee).

Synthesis of (4S)-trans-,cis-[2-(4-chlorophenyl)-2-bromomethyl-1,3-dioxolan-4-yl]methyl methanesulfate

To a mixture of (4R)-trans-,cis-2-(4-chlorophenyl)-2-bromomethyl-1,3-dioxolan-4-methanol (2.04 g), triethylamine (0.89 g, 1.5 equivalents) and methylene chloride (67 mL) was added mesyl chloride (0.80 g, 1.2 equivalents) at 5˚C, and the mixture was stirred for 5 hr. The reaction mixture was washed with water, and the solvent was evaporated under reduced pressure to give crude (4S)-trans-,cis-[2-(4-chlorophenyl)-2-bromomethyl-1,3-dioxolan-4-yl]methyl methanesulfonate (2.41 g, >99%ee).

Synthesis of (4S)-cis-[2-(4-chlorophenyl)-2-bromomethyl-1,3-dioxolan-4-yl]methyl methanesulfonate

To crude (4S)-trans-,cis-[2-(4-chlorophenyl)-2-bromomethyl-1,3-dioxolan-4-yl]methyl methanesulfonate (2.41 g) was added methanol (44 mL) to allow recrystallization at room temperature to give (4S)-cis-[2-(4-chlorophenyl)-2-bromomethyl-1,3-dioxolan-4-yl]methyl methanesulfonate as colorless crystals (0.67 g, >99%ee). Here, the content percentage of (4S)-cis-[2-(4-chlorophenyl)-2-chloromethyl-1,3-dioxolan-4-yl]methyl methanesulfonate, which was derived from the halogen-exchanged compound in Example 1, was also 1.3%.

Examples 53 to 55

Synthesis of (4S)-cis-[2-aryl-2-bromomethyl-1,3-dioxolan-4-yl]methyl methanesulfonate

In Examples 53 to 55, reactions were performed according to Example 52 and using (4S)-trans-,cis-[2-aryl-2-bromomethyl-1,3-dioxolan-4-yl]methyl benzoates in Table 14, which were synthesized in Examples 49 to 51. The results before recrystallization are shown in Table 15, and the results after recrystallization are shown in Table 16 together with Example 52.

[Table 14]

| Example | substrate | | | cis/trans | halogen-exchanged compound content (%) |
|---|---|---|---|---|---|
| | cis | trans | halogen-exchanged compound | | |
| 52 | 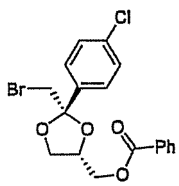 | 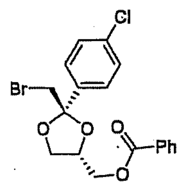 | 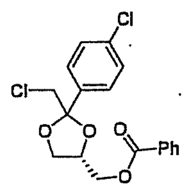 | 66/34 | 1. 3 |
| 53 | 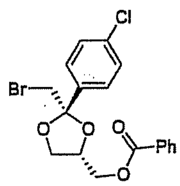 | 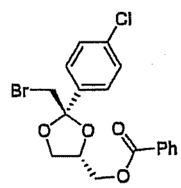 | 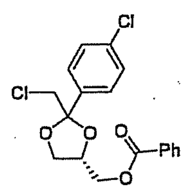 | 66/34 | 0.09 |
| 54 | 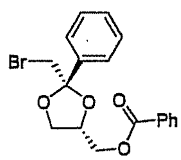 | 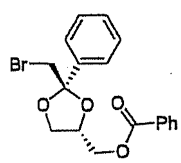 | 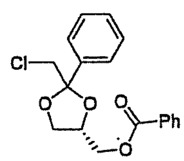 | 66/34 | 1. 3 |
| 55 | 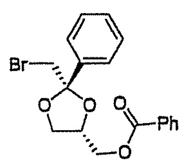 | 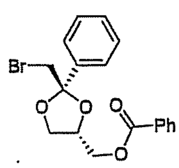 | 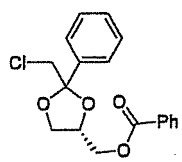 | 66/34 | 0.06 |

[Table 15]

| Example | desired product | | halogen-exchanged compound | yield (%) of desired product | cis/trans | halogen-exchanged compound content (%) |
|---|---|---|---|---|---|---|
| | cis | trans | | | | |
| 52 | 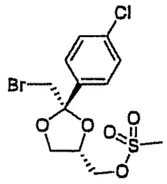 | 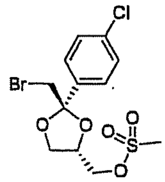 | 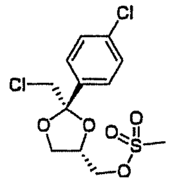 | 96 | 66/34 | 1.3 |
| 53 | 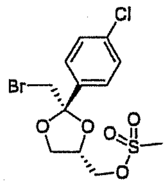 | 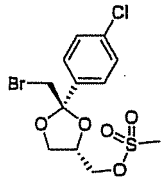 | 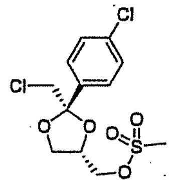 | 96 | 66/34 | 0.09 |
| 54 | 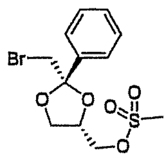 | 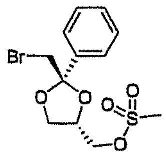 | 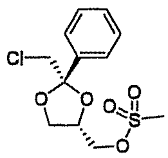 | 94 | 66/34 | 1.3 |
| 55 | 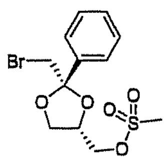 | 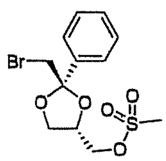 | 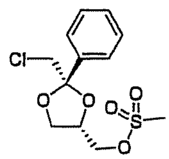 | 95 | 66/34 | 0.06 |

[Table 16]

| Example | desired product (cis) | halogen-exchanged compound | yield (%) of desired product | cis/trans | halogen-exchanged compound content (%) |
|---|---|---|---|---|---|
| 52 | 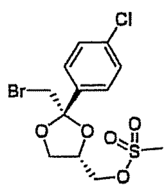 | 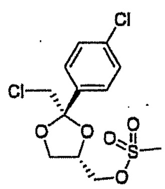 | 26 | >99/<1 | 1.3 |
| 53 | 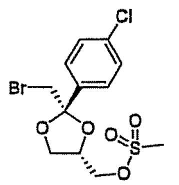 | 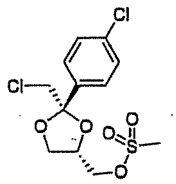 | 28 | >99/<1 | 0.09 |

(continued)

| Example | desired product (cis) | halogen-exchanged compound | yield (%) of desired product | cis/trans | halogen-exchanged compound content (%) |
|---|---|---|---|---|---|
| 54 | 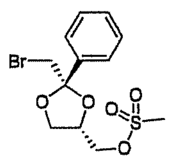 | 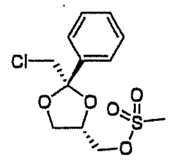 | 25 | >99/<1 | 1.3 |
| 55 | 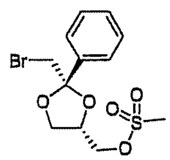 | 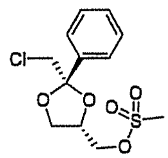 | 29 | >99/<1 | 0.06 |

Example 56

Synthesis of (4S)-cis-[2-(4-chlorophenyl)-2-bromomethyl-1,3-dioxolan-4-yl]methyl benzoate

To (4S)-trans-,cis-[2-(4-chlorophenyl)-2-bromomethyl-1,3-dioxolan-4-yl]methyl benzoate (2.61 g) synthesized in Example 42 was added methanol (30 mL) to allow recrystallization at room temperature to give (4S)-cis-[2-(4-chlorophenyl)-2-bromomethyl-1,3-dioxolan-4-yl]methyl benzoate as colorless crystals (1.23 g, >99%ee). The content percentage of (4S)-cis-[2-(4-chlorophenyl)-2-chloromethyl-1,3-dioxolan-4-yl]methyl benzoate, which was derived from the halogen-exchanged compound in Example 1, was 1.3%.

Examples 57 to 59

Synthesis of (4S)-cis-[2-aryl-2-bromomethyl-1,3-dioxolan-4-yl]methyl benzoate

In Examples 57 to 59, recrystallization was performed according to Example 56 and using (4S)-trans-,cis-[2-aryl-2-halomethyl-1,3-dioxolan-4-yl]methyl benzoates in Table 17, which were synthesized in Examples 49 to 51. The results are shown in Table 18 together with Example 56.

[Table 17]

| Example | substrate | | | cis/trans | halogen-exchanged compound content (%) |
|---|---|---|---|---|---|
| | cis | trans | halogen-exchanged compound | | |
| 56 | 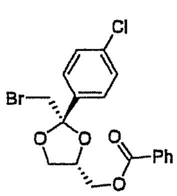 | 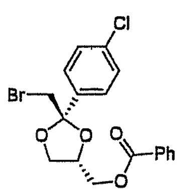 | 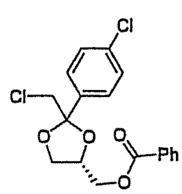 | 66/34 | 1. 3 |
| 57 | 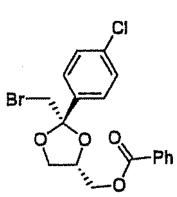 | 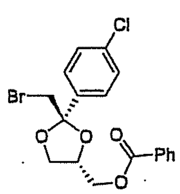 | 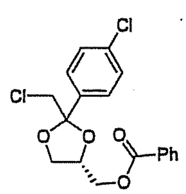 | 66/34 | 0.09 |

(continued)

| Example | substrate | | | cis/trans | halogen-exchanged compound content (%) |
|---|---|---|---|---|---|
| | cis | trans | halogen-exchanged compound | | |
| 58 | 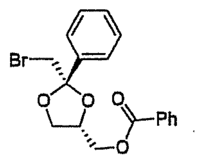 | 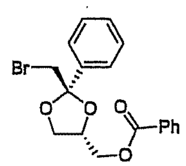 | 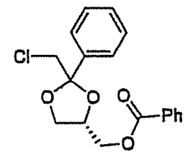 | 66/34 | 1. 3 |
| 59 | 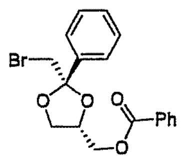 | 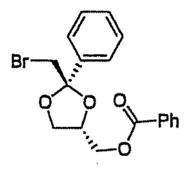 | 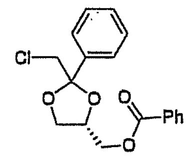 | 66/34 | 0.06 |

[Table 18]

| Example | desired product (cis) | halogen-exchanged compound | yield (%) of desired product | cis/trans | halogen-exchanged compound content (%) |
|---|---|---|---|---|---|
| 56 | 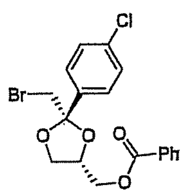 | 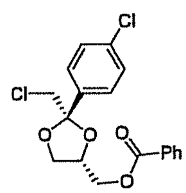 | 47 | >99/<1 | 1.3 |
| 57 | 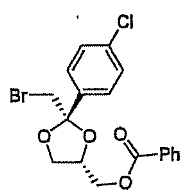 | 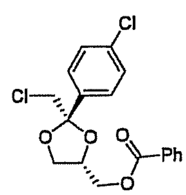 | 47 | >99/<1 | 0.09 |
| 58 | 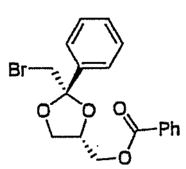 | 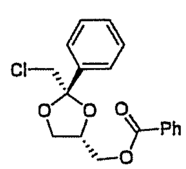 | 50 | >99/<1 | 1.3 |
| 59 | 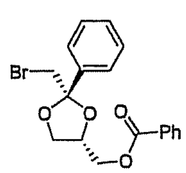 | 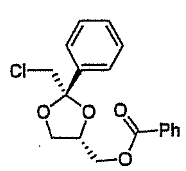 | 46 | >99/<1 | 0.06 |

In Examples 1 to 16, reactions were performed after mixing the entire amounts of various aryl(halomethyl)ketones with monochlorohydrin. As shown in Examples 1 to 12, when $X^1$ is other than a chlorine atom, the content of the halogen-exchanged compound increased, but the object optically active 2-aryl-2-halomethyl-4-chloromethyl-1,3-dioxolane could be synthesized without decreasing the optical purity. When $X^1$ is a chlorine atom as in Examples 13 to 16, a halogen-exchanged compound was not produced, and optically active 2-aryl-2-chloromethyl-4-chloromethyl-1,3-dioxolane was obtained without decreasing the optical purity.

Examples 17 to 22 show the results of synthesis of optically active 2-aryl-2-halomethyl-4-chloromethyl-1,3-dioxolane by changing to various acid catalysts. Examples 23 to 41 show the results of shortened contact time with aryl (halomethyl)ketone by dropwise addition of monochlorohydrin, where an increase in the content of halogen-exchanged compound could be controlled. When an acid catalyst that extricates a halide ion wherein $X^1 \neq X^2$ was used as in Example 28, halogen exchange occurred with the catalyst, and the content of halogen-exchanged compound increased.

Examples 42 to 51 show the results of induction of the obtained optically active 2-aryl-2-chloromethyl-4-chloromethyl-1,3-dioxolane to optically active (2-aryl-2-halomethyl-1,3-dioxolan-4-yl)methyl carboxylate, and Examples 52 to 55 show the results of induction to optically active (2-aryl-2-halomethyl-1,3-dioxolan-4-yl)methyl sulfonate, followed by recrystallization to isolate optically active cis-(2-aryl-2-halomethyl-1,3-dioxolan-4-yl)methyl sulfonate. Examples 56 to 59 show the results of recrystallization at the stage of optically active (2-aryl-2-halomethyl-1,3-dioxolan-4-yl)methyl carboxylate to isolate optically active cis-(2-aryl-2-halomethyl-1,3-dioxolan-4-yl)methyl carboxylate. In Examples 42 to 59, it is appreciated that the halogen-exchanged compound content percentage does not change much before and after the reaction, and control during the production of optically active 2-aryl-2-halomethyl-4-chloromethyl-1,3-dioxolane is important.

While the isomerization step of the present invention is explained by referring to Examples, which are not to be construed as limitative. The isolation yield of the cis form after recrystallization is about 80% based on the quantification yield of cis form by high performance liquid chromatography after isomerization.

Example 60

Isomerizationof(4S)-trans-[2-(4-chlorophenyl)-2-bromomethyl-1,3-dioxolan-4-yl]methylbenzoateto(4S)-cis-[2-(4-chlorophenyl)-2-bromomethyl-1,3-dioxolan-4-yl]methyl benzoate

(4S)-[2-(4-Chlorophenyl)-2-bromomethyl-1,3-dioxolan-4-yl]methyl benzoate (2.0 g, 4.86 mmol, cis form:trans form=27:73) was dissolved in toluene (5 mL) as a solvent, a boron trifluoride-diethyl ether complex (0.34 g, 2.43 mmol) as a catalyst was added thereto, and the mixture was stirred at 25°C for 1 hr. When the mixture at this stage was measured by high performance liquid chromatography (apparatus used: SHIMADZU Corporation LC-9A, column used: DAISOPAK SP-120-5-ODS-BP (Daiso Co., Ltd.), the quantification yield was 46% for the cis form, and 44% for the trans form (cis form:trans form=51:49). Thereafter, the reaction mixture was neutralized with triethylamine, and concentrated to dryness with a rotary evaporator. The residue was dissolved in methanol, and the mixture was stirred to allow precipitation of crystals of the cis form. The precipitated crystals were collected by filtration and dried to give (4S)-cis-[2-(4-chlorophenyl)-2-bromomethyl-1,3-dioxolan-4-yl]methyl benzoate (0.72 g, isolation yield 36%).

Examples 61 to 72

In Examples 61 to 72, isomerization was performed according to Example 60 under the reaction conditions of Table 19 and Table 20, and optically active cis-(2-aryl-2-halomethyl-1,3-dioxolan-4-yl)methyl carboxylate was isolated. While only the trans forms are shown in the Tables, the substrate used was a mixture of the cis form and the trans form at the ratio of (). The results are shown in Table 21.

[Table 19]

| Example | substrate | | catalyst | solvent | reaction time, reaction temperature |
|---|---|---|---|---|---|
| | substrate (cis form: trans form) | substrate amount | | | |
| 60 | 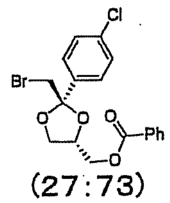 (27:73) | 4. 86mmol | $BF_3$- $Et_2O$ (2.43mmol) | toluene (5mL) | 1h .25˚C |
| 61 | 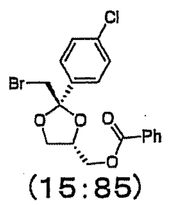 (15:85) | 4. 86mmol | $AlCl_3$ (4.86mmol) | $CH_2Cl_2$ (10mL) | 12h .25˚C |
| 62 | 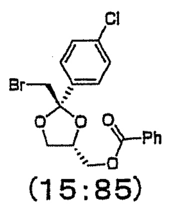 (15:85) | 4. 86mmol | $SnCl_2$ (4.86mmol) | $CH_2Cl_2$ (10mL) | 12h .25˚C |
| 63 | 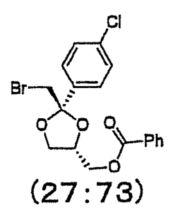 (27:73) | 4. 86mmol | MsOH (4.86mmol) | $CH_2Cl_2$ (10mL) | 3h .25˚C |
| 64 | 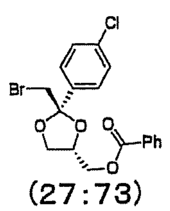 (27:73) | 4. 86mmol | HCl (77.8mmol) | EtOAc (20mL) | 4h .25˚C |
| 65 | 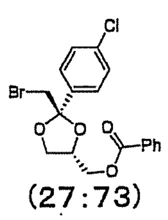 (27:73) | 4. 86mmol | TFA (136.1mmol) | -- | 4h .25˚G |

(continued)

| Example | substrate | | catalyst | solvent | reaction time, reaction temperature |
|---|---|---|---|---|---|
| | substrate (cis form: trans form) | substrate amount | | | |
| 66 | 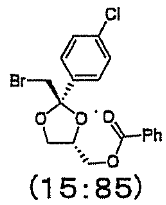 (15:85) | 4. 86mmol | $BF_3 \cdot Et_2O$ (4.86mmo) | $CH_3CN$ (10mL) | 36h .25˚C |

[Table 20]

| Example | substrate | | catalyst | solvent | reaction time, reaction temperature |
|---|---|---|---|---|---|
| | substrate (cis form: trans form) | substrate amount | | | |
| 67 | 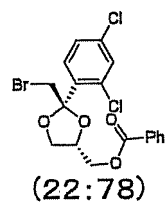 (22:78) | 4. 48mmol | $BF_3.Et_2O$ (2.24mmol) | toluene: heptane = 9:1 (5mL) | 2h .25˚C |
| 68 | 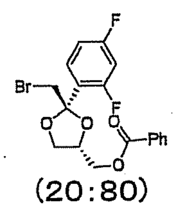 (20:80) | 4. 84mmol | $BF_3 \cdot Et_2O$ (2.42mmol) | toluene (5mL) | 2h .25˚C |
| 69 | 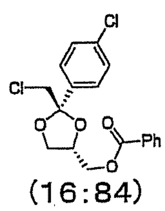 (16:84) | 5. 45mmol | $BF_3 \cdot Et_2O$ (2.73mmol) | toluene (5mL) | 2h .25˚C |
| 70 | 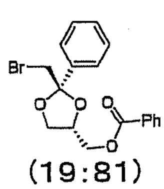 (19:81) | 5. 30mmol | $BF_3.Et_2O$ (2.65mmol) | toluene (5mL) | 2h .25˚C |

(continued)

| Example | substrate | | catalyst | solvent | reaction time, reaction temperature |
|---|---|---|---|---|---|
| | substrate (cis form: trans form) | substrate amount | | | |
| 71 | 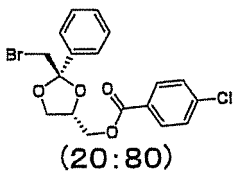 (20:80) | 4. 86mmol | $BF_3 \cdot Et_2O$ (2.43mmol) | toluene (5mL) | 2h .25°C |
| 72 | 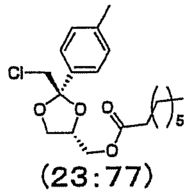 (23:77) | 5. 42mmol | $BF_3 \cdot Et_2O$ (2.71mmol) | toluene (5mL) | 2h .25°C |

[Table 21]

| Example | before isomerization | after isomerization | after recrystallization |
|---|---|---|---|
| | yield, quantitative cis form, trans form | yield, quantitative cis form, trans form | cis form isolation yield |
| 60 | 27%,73% | 46%,44% | 36% |
| 61 | 15%,85% | 45%,42% | 35% |
| 62 | 15%,85% | 41%,46% | 33% |
| 63 | 27%,73% | 38%,35% | 31% |
| 64 | 15%,85% | 29%,34% | 23% |
| 65 | 15%,85% | 33%,39% | 25% |
| 66 | 15%,85% | 35%,38% | 28% |
| 67 | 22%,78% | 46%,46% | 37% |
| 68 | 20%,80% | 42%,46% | 33% |
| 69 | 16%,84% | 45%,44% | 36% |
| 70 | 19%,81% | 44%,45% | 36% |
| 71 | 20%,80% | 43%,43% | 35% |
| 72 | 23%,77% | 45%,45% | 36% |

Example 73

Isomerization of (4S)-trans-[2-(4-chlorophenyl)-2-bromomethyl-1,3-dioxolan-4-yl]methyl benzoate while crystallizing (4S)-cis-[2-(4-chlorophenyl)-2-bromomethyl-1,3-dioxolan-4-yl]methyl benzoate

(4S)-[2-(4-Chlorophenyl)-2-bromomethyl-1,3-dioxolan-4-yl]methyl benzoate (20 g, 48.6 mmol, cis form:trans form=27:73) was dissolved in toluene (20 mL) as a solvent, and a boron trifluoride-diethyl ether complex (3.4 g, 24.3 mmol) as a catalyst was added thereto. The atmospheric pressure was reduced to 35 mmHg, and the mixture was

stirred for 3 hr while evaporating the solvent to precipitate crystals. The reaction mixture was quenched with triethylamine, and the crystals were dissolved by adding toluene. The solution was analyzed by high performance liquid chromatography in the same manner as in Example 60, and the quantification yield was 78.5% for the cis form and 13% for the trans form (cis form:trans form=86:14). This solution was concentrated to dryness with a rotary evaporator, the residue was dissolved in methanol, and the mixture was stirred to allow precipitation of crystals of the cis form. The precipitated crystals were collected by filtration and dried to give (4S)-cis-[2-(4-chlorophenyl)-2-bromomethyl-1,3-dioxolan-4-yl]methyl benzoate (13.3 g, isolation yield 66.5%).

It is appreciated from the results of Examples 60 to 72 that isomerization improves the quantification yield of cis form. Example 73 shows the results of isomerization while precipitating crystals, where the quantification yield of the cis form increased markedly.

Example 74

Crystallization of (4S)-cis-[2-(4-chlorophenyl)-2-bromomethyl-1,3-dioxolan-4-yl]methyl benzoate while isomerizing (4S)-trans-[2-(4-chlorophenyl)-2-bromomethyl-1,3-dioxolan-4-yl]methyl benzoate continuously from the synthesis of (4S)-trans-,cis-[2-(4-chlorophenyl)-2-bromomethyl-1,3-dioxolan-4-yl]methyl benzoate

A mixture of (4S)-trans-,cis-2-(4-chlorophehyl)-2-bromomethyl-4-chloromethyl-1,3-dioxolane (6.36 g) synthesized in Example 1, sodium benzoate (2.88 g, 1.0 equivalent) and DMF (100 mL) was stirred with heating at 150˚C for 8 hr. After cooling, the reaction mixture was diluted with water (100 mL), and extracted 3 times with ethyl acetate (100 mL). The organic layer was washed with water, and the solvent was evaporated under reduced pressure. The residue was dissolved in heptane (50 mL) at 50˚C. Thereto was added a boron trifluoride-diethyl ether complex (2.84 g, 1.0 equivalent) as a catalyst, and the mixture was cooled to 0˚C to allow crystal precipitation while isomerizing. The reaction mixture was quenched with triethylamine, and recrystallized to give (4S)-cis-[2-(4-chlorophenyl)-2-bromomethyl-1,3-dioxolan-4-yl]methyl benzoate (4.9 g, >99%ee, cis form:trans form=>99:<1, yield 61%).

Example 75

Synthesis of (4R)-cis-2-(4-chlorophenyl)-2-bromomethyl-1,3-dioxolan-4-methanol

To a mixture of (4S)-cis-[2-(4-chlorophenyl)-2-bromomethyl-1,3-dioxolan-4-yl]methyl benzoate (2.73 g, cis form:trans form=>99:<1) synthesized in Example 60 and dioxane (67 mL) was added 10% NaOH (4.00 g, 1.5 equivalents) at 5˚C, and the mixture was stirred for 5 hr. The reaction mixture was diluted with water (67 mL), and extracted 3 times with ethyl acetate (67 mL). The organic layer was washed with water, and the solvent was evaporated under reduced pressure to give (4R)-cis-2-(4-chlorophenyl)-2-bromoinethyl-1,3-dioxolan-4-methanol (2.04 g, >99%ee).

Synthesis of (4S)-cis-[2-(4-chlorophenyl)-2-bromomethyl-1,3-dioxolan-4-yl]methyl methanesulfonate

To a mixture of (4R)-cis-2-(4-chlorophenyl)-2-bromomethyl-1,3-dioxolan-4-methanol (2.04 g), triethylamine (0.89 g, 1.5 equivalents) and methylene chloride (67 mL) was added mesyl chloride (0.80 g, 1.2 equivalents) at 5˚C, and the mixture was stirred for 5 hr. The reaction mixture was washed with water, and the solvent was evaporated under reduced pressure. Methanol (44 mL) was added, and the mixture was recrystallized at room temperature to give (4S)-cis-[2-(4-chlorophenyl)-2-bromomethyl-1,3-dioxolan-4-yl]methyl methanesulfonate as colorless crystals (2.22 g, >99%ee, cis form:trans form=>99:<1, yield 86%).

Examples 76 to 78

Synthesis of (4S)-cis-[2-aryl-2-bromomethyl-1,3-dioxolan-4-yl]methyl methanesulfonate

In Examples 76 to 78, reactions were performed according to Example 75 and using (4S)-cis-[2-aryl-2-bromomethyl-1,3-dioxolan-4-yl]methyl benzoates synthesized in Examples 67, 68 and 70. The results are shown in Table 22 together with Example 75.

[Table 22]

| Example | substrate (cis) | substrate cis/ trans | desired product (cis) | yield (%) of desired product | desired product cis/trans |
|---|---|---|---|---|---|
| 75 | 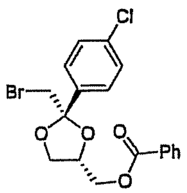 | >99/<1 | 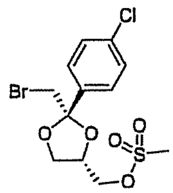 | 86 | >99/<1 |
| 76 | 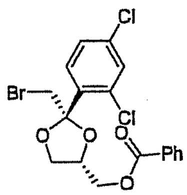 | >99/<1 | 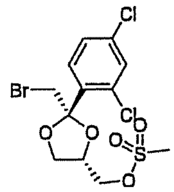 | 84 | >99/<1 |
| 77 | 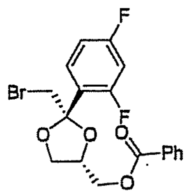 | >99/<1 | 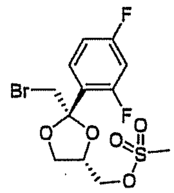 | 87 | >99/<1 |
| 78 | 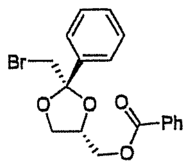 | >99/<1 | 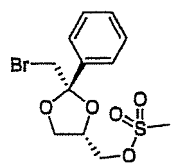 | 87 | >99/<1 |

Industrial Applicability

According to the present invention, optically active 2-aryl-2-halomethyl-4-chloromethyl-1,3-dioxolane can be constructed from easily available, economical optically active monochlorohydrin as a starting material, without decreasing the optical purity. Optically active (2-aryl-2-halomethyl-1,3-dioxolan-4-yl)methyl carboxylate can be produced from the obtained optically active 2-aryl-2-halomethyl-4-chloromethyl-1,3-dioxolane, without decreasing the optical purity. In addition, it can also be induced to further useful optically active (2-aryl-2-halomethyl-1,3-dioxolan-4-yl)methyl sulfonate having a high chemical purity.

Moreover, the present invention can isomerize the optically active trans-(2-aryl-2-halomethyl-1,3-dioxolan-4-yl)methyl carboxylate to efficiently lead same to optically active cis-(2-aryl-2-halomethyl-1,3-dioxolan-4-yl)methyl carboxylate useful as an intermediate for ketoconazole (an antifungal agent) and the like.

The thus-obtained optically active (2-aryl-2-halomethyl-1,3-dioxolan-4-yl)methyl carboxylate and optically active (2-aryl-2-halomethyl-1,3-dioxolan-4-yl)methyl sulfonate can be used as various pharmaceutical intermediates for ketoconazole (an antifungal agent) and the like.

This application is based on patent applications Nos. 200225/2008 and 235746/2008 filed in Japan, the contents of which are hereby incorporated in their entireties.

Although the present invention have been presented or described by referring to preferred embodiments of this invention, it will, however, be understood by those of ordinary skill in the art that various modifications may be made to the forms and details without departing from the scope of the invention as set forth in the appended claims. All patents, patent publications and other publications indicated or cited in the Specification are hereby incorporated in their entireties by reference.

**Claims**

1. A method of producing (2R,trans)-2-aryl-2-halomethyl-4-chloromethyl-1,3-dioxolane represented by the formula [III]

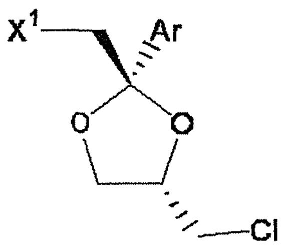

[III]

wherein Ar is a substituted or unsubstituted aromatic ring group, or a substituted or unsubstituted heteroaromatic ring group, and $X^1$ is a halogen atom, and/or (2S,cis)-2-aryl-2-halomethyl-4-chloromethyl-1,3-dioxolane represented by the formula [IV]

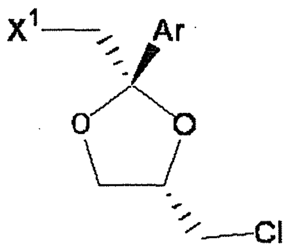

[IV]

wherein each symbol is as defined above, comprising reacting aryl(halomethyl)ketone represented by the formula [I]

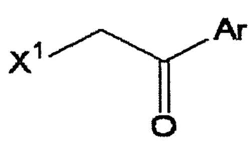

[I]

wherein each symbol is as defined above, with (S)-monochlorohydrin represented by the formula [II]

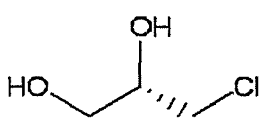

[II]

in the presence of an acid catalyst.

2. The production method according to claim 1, wherein $X^1$ is a halogen atom other than a chlorine atom, and (S)-monochlorohydrin represented by the formula [II] is added to aryl(halomethyl)ketone represented by the formula [I].

3. The production method according to claim 2, wherein (S)-monochlorohydrin is added such that the abundance of (S)-monochlorohydrin represented by the formula [II] during the reaction is not more than 0.2 equivalent of the amount used of aryl(halomethyl)ketone represented by the formula [I].

4. The production method according to claim 2 or 3, wherein (S)-monochlorohydrin is added such that the abundance of (S)-monochlorohydrin represented by the formula [II] during the reaction is not more than 0.1 equivalent of the amount used of aryl(halomethyl)ketone represented by the formula [I].

5. The production method according to any of claims 1 to 4, wherein an acid catalyst that does not extricate a halide ion other than $X^1$ is used as the acid catalyst.

6. A method of producing (2R,trans)-(2-aryl-2-halomethyl-1,3-dioxolan-4-yl)methyl carboxylate represented by the formula [V]

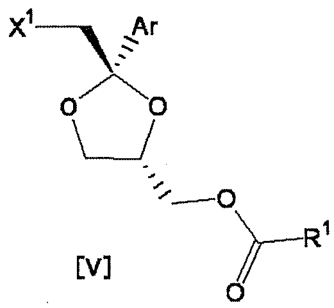

[V]

wherein Ar is a substituted or unsubstituted aromatic ring group, or a substituted or unsubstituted heteroaromatic ring group, $X^1$ is a halogen atom, and $R^1$ is a substituted or unsubstituted hydrocarbon group, or a substituted or unsubstituted aromatic ring group, and/or (2S,cis)-(2-aryl-2-halomethyl-1,3-dioxolan-4-yl)methyl carboxylate represented by the formula [VI]

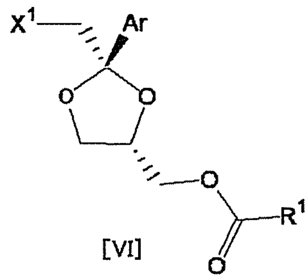

[VI]

wherein each symbol is as defined above, comprising converting (2R,trans)-2-aryl-2-halomethyl-4-chloromethyl-1,3-dioxolane represented by the formula [III] and/or (2S,cis)-2-aryl-2-halomethyl-4-chloromethyl-1,3-dioxolane represented by the formula [IV] obtained by the production method according to any of claims 1 to 5 to (2R,trans)-(2-aryl-2-halomethyl-1,3-dioxolan-4-yl)methyl carboxylate and/or (2S,cis)-(2-aryl-2-halomethyl-1,3-dioxolan-4-yl)methyl carboxylate.

7. A method of producing (2S,cis)-(2-aryl-2-halomethyl-1,3-dioxolan-4-yl)methyl sulfonate represented by the formula [X]

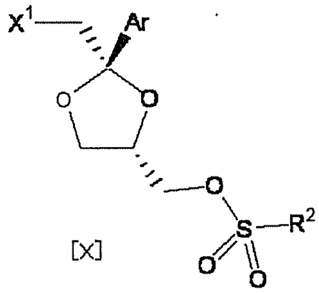

[X]

wherein Ar is a substituted or unsubstituted aromatic ring group, or a substituted or unsubstituted heteroaromatic ring group, $X^1$ is a halogen atom, and $R^2$ is a substituted or unsubstituted hydrocarbon group, or a substituted or unsubstituted aromatic ring group,
comprising a step of hydrolyzing (2R,trans)-(2-aryl-2-halomethyl-1,3-dioxolan-4-yl)methyl carboxylate represented by the formula [V] and/or (2S,cis)-(2-aryl-2-halomethyl-1,3-dioxolan-4-yl)methyl carboxylate represented by the formula [VI], which are obtained by the production method according to claim 6 to give (2R,trans)-2-aryl-2-halomethyl-1,3-dioxolan-4-methanol represented by the formula [VII]

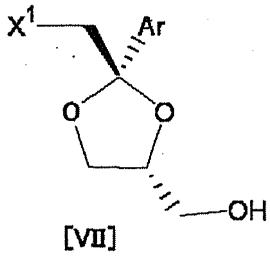

[VII]

wherein each symbol is as defined above, and/or (2S,cis)-2-aryl-2-halomethyl-1,3-dioxolan-4-methanol represented by the formula [VIII]

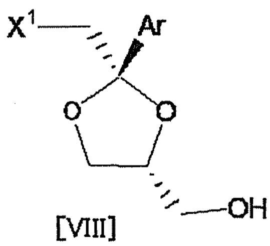

[VIII]

wherein each symbol is as defined above,
a step of converting (2R,trans)-2-aryl-2-halomethyl-1,3-dioxolan-4-methanol represented by the formula [VII] and/or (2S,cis)-2-aryl-2-halomethyl-1,3-dioxolan-4-methanol represented by the formula [VIII] to (2R,trans)-(2-aryl-2-halomethyl-1,3-dioxolan-4-yl)methyl sulfonate represented by the formula [IX]

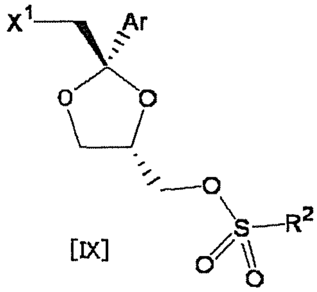

[IX]

wherein each symbol is as defined above, and/or (2S,cis)-(2-aryl-2-halomethyl-1,3-dioxolan-4-yl)methyl sulfonate represented by the formula [X], and a step of isolating (2S,cis)-(2-aryl-2-halomethyl-1,3-dioxolan-4-yl)methyl sulfonate represented by the formula [X] by recrystallization from a mixture containing (2R,trans)-(2-aryl-2-halomethyl-1,3-dioxolan-4-yl)methyl sulfonate represented by the formula [IX] and (2S,cis)-(2-aryl-2-halomethyl-1,3-dioxolan-4-yl)methyl sulfonate represented by the formula [X].

**8.** A method of producing (2S,cis)-(2-aryl-2-halomethyl-1,3-dioxolan-4-yl)methyl carboxylate represented by the formula [VI], wherein (2S,cis)-(2-aryl-2-halomethyl-1,3-dioxolan-4-yl)methyl carboxylate represented by the formula [VI] is isolated by recrystallization from a mixture containing (2R,trans)-(2-aryl-2-halomethyl-1,3-dioxolan-4-yl)methyl carboxylate represented by the formula [V] and (2S,cis)-(2-aryl-2-halomethyl-1,3-dioxolan-4-yl)methyl carboxylate represented by the formula [VI], which are obtained by the production method according to claim 6.

**9.** A method of producing (2S,cis)-(2-aryl-2-halomethyl-1,3-dioxolan-4-yl)methyl carboxylate represented by the formula [VI], comprising isomerizing (2R,trans)-(2-aryl-2-halomethyl-1,3-dioxolan-4-yl)methyl carboxylate represented by the formula [V], which is contained in the mother liquid of the recrystallization in the method according to claim 8, in the presence of an acid catalyst.

**10.** The production method according to claim 9, which is performed in at least one kind of solvent selected from aliphatic hydrocarbon solvents, aromatic hydrocarbon solvents, halogenated hydrocarbon solvents, nitrile solvents, ester solvents and acid catalysts.

**11.** The production method according to claim 9 or 10, comprising a step of isolating (2S,cis)-(2-aryl-2-halomethyl-1,3-dioxolan-4-yl)methyl carboxylate represented by the formula [VI] after the isomerization step.

**12.** The production method according to claim 11, comprising isolation by recrystallization.

**13.** A method of producing (2S,cis)-(2-aryl-2-halomethyl-1,3-dioxolan-4-yl)methyl carboxylate represented by the formula [VI], comprising reacting a mixture containing (2R,trans)-(2-aryl-2-halomethyl-1,3-dioxolan-4-yl)methyl carboxylate represented by the formula [V] and (2S,cis)-(2-aryl-2-halomethyl-1,3-dioxolan-4-yl)methyl carboxylate represented by the formula [VI], which are obtained by the production method according to claim 6, in the presence of an acid catalyst to isomerize (2R,trans)-(2-aryl-2-halomethyl-1,3-dioxolan-4-yl)methyl carboxylate represented by the formula [V] to (2S,cis)-(2-aryl-2-halomethyl-1,3-dioxolan-4-yl)methyl carboxylate represented by the formula [VI], while simultaneously precipitating (2S,cis)-(2-aryl-2-halomethyl-1,3-dioxolan-4-yl)methyl carboxylate represented by the formula [VI] as crystals.

**14.** The production method according to claim 13, which is performed in at least one kind of solvent selected from aliphatic hydrocarbon solvents, aromatic hydrocarbon solvents, halogenated hydrocarbon solvents, nitrile solvents, ester solvents and acid catalysts.

**15.** The production method according to any of claims 9 to 14, wherein the acid catalyst is a Lewis acid or an organic acid.

**16.** A method of producing (2S,cis)-(2-aryl-2-halomethyl-1,3-dioxolan-4-yl)methyl sulfonate represented by the formula [X]

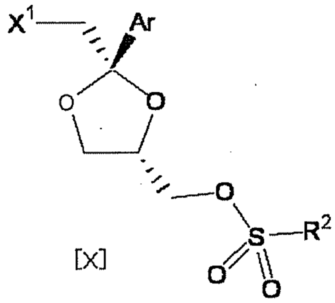

[X]

wherein Ar is a substituted or unsubstituted aromatic ring group, or a substituted or unsubstituted heteroaromatic ring group, and $X^1$ is a halogen atom, comprising a step of hydrolyzing (2S,cis)-(2-aryl-2-halomethyl-1,3-dioxolan-4-yl)methyl carboxylate represented by the formula [VI] obtained by the production method according to any of claims 8 to 15 to give (2S,cis)-2-aryl-2-halomethyl-1,3-dioxolan-4-methanol represented by the formula [VIII]

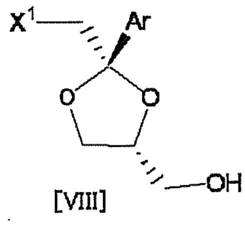

[VIII]

wherein each symbol is as defined above, and
a step of converting (2S,cis)-2-aryl-2-halomethyl-1,3-dioxolan-4-methanol represented by the formula [VIII] to (2S, cis)-(2-aryl-2-halomethyl-1,3-dioxolan-4-yl)methyl sulfonate represented by the formula [X].

**17.** A method of producing (2S,trans)-2-aryl-2-halomethyl-4-chloromethyl-1,3-dioxolane represented by the formula [XII]

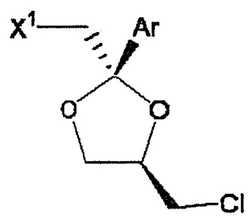

[XII]

wherein Ar is a substituted or unsubstituted aromatic ring group, or a substituted or unsubstituted heteroaromatic ring group, and $X^1$ is a halogen atom, and/or (2R,cis)-2-aryl-2-halomethyl-4-chloromethyl-1,3-dioxolane represented by the formula [XIII]

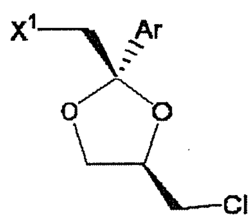

[XIII]

wherein each symbol is as defined above, comprising reacting aryl(halomethyl)ketone represented by the formula [I]

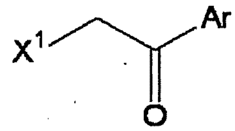

[I]

wherein each symbol is as defined above, with (R)-monochlorohydrin represented by the formula [XI]

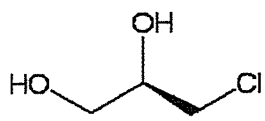

[XI]

in the presence of an acid catalyst.

**18.** The production method according to claim 17, wherein $X^1$ is a halogen atom other than a chlorine atom, and (R)-monochlorohydrin represented by the formula [XI] is added to aryl(halomethyl)ketone represented by the formula [I].

**19.** The production method according to claim 18, wherein (R)-monochlorohydrin is added such that the abundance of (R)-monochlorohydrin represented by the formula [XI] during the reaction is not more than 0.2 equivalent of the amount used of aryl(halomethyl)ketone represented by the formula [I].

**20.** The production method according to claim 18 or 19, wherein (R)-monochlorohydrin is added such that the abundance of (R)-monochlorohydrin represented by the formula [XI] during the reaction is not more than 0.1 equivalent of the amount used of aryl(halomethyl)ketone represented by the formula [I].

**21.** The production method according to any of claims 17 to 20, wherein an acid catalyst that does not extricate a halide ion other than $X^1$ is used as the acid catalyst.

**22.** A method of producing (2S,trans)-(2-aryl-2-halomethyl-1,3-dioxolan-4-yl)methyl carboxylate represented by the formula [XIV]

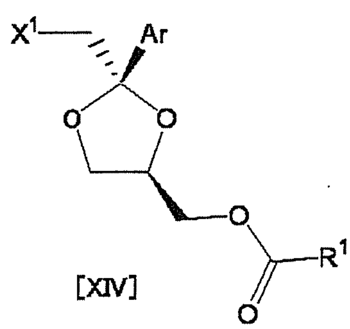

[XIV]

wherein Ar is a substituted or unsubstituted aromatic ring group, or a substituted or unsubstituted heteroaromatic ring group, $X^1$ is a halogen atom, and $R^1$ is a substituted or unsubstituted hydrocarbon group, or a substituted or unsubstituted aromatic ring group, and/or (2R,cis)-(2-aryl-2-halomethyl-1,3-dioxolan-4-yl)methyl carboxylate represented by the formula [XV]

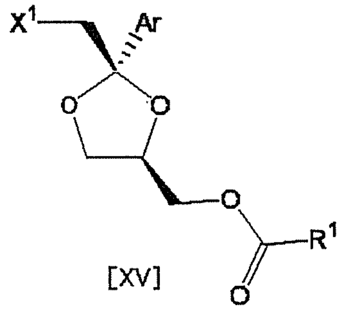

[XV]

wherein each symbol is as defined above, comprising converting (2S,trans)-2-aryl-2-halomethyl-4-chloromethyl-1,3-dioxolane represented by the formula [XII] and/or (2R,cis)-2-aryl-2-halomethyl-4-chloromethyl-1,3-dioxolane represented by the formula [XIII], which are obtained by the production method according to any of claims 17 to 21, to (2S,trans)-(2-aryl-2-halomethyl-1,3-dioxolan-4-yl)methyl carboxylate, and/or (2R,cis)-(2-aryl-2-halomethyl-1,3-dioxolan-4-yl)methyl carboxylate.

**23.** A method of producing (2R,cis)-(2-aryl-2-halomethyl-1,3-dioxolan-4-yl)methyl sulfonate represented by the formula [XIX]

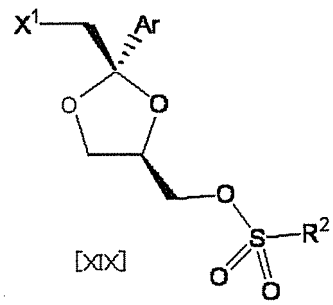

[XIX]

wherein Ar is a substituted or unsubstituted aromatic ring group, or a substituted or unsubstituted heteroaromatic ring group, $X^1$ is a halogen atom, and $R^2$ is a substituted or unsubstituted hydrocarbon group, or a substituted or unsubstituted aromatic ring group,
comprising hydrolyzing (2S,trans)-(2-aryl-2-halomethyl-1,3-dioxolan-4-yl)methyl carboxylate represented by the formula [XIV] and/or (2R,cis)-(2-aryl-2-halomethyl-1,3-dioxolan-4-yl)methyl carboxylate represented by the formula [XV], which are obtained by the production method according to claim 22, to give (2S,trans)-2-aryl-2-halomethyl-1,3-dioxolan-4-methanol represented by the formula [XVI]

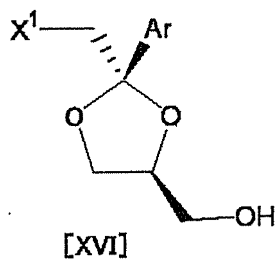

[XVI]

wherein each symbol is as defined above, and/or (2R,cis)-2-aryl-2-halomethyl-1,3-dioxolan-4-methanol represented by the formula [XVII]

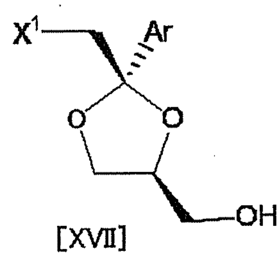

[XVII]

wherein each symbol is as defined above,
a step of converting (2S,trans)-2-aryl-2-halomethyl-1,3-dioxolan-4-methanol represented by the formula [XVI] and/or (2R,cis)-2-aryl-2-halomethyl-1,3-dioxolan-4-methanol represented by the formula [XVII] to (2S,trans)-(2-aryl-2-halomethyl-1,3-dioxolan-4-yl)methyl sulfonate represented by the formula [XVIII]

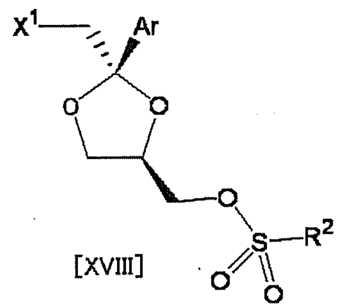

[XVIII]

wherein each symbol is as defined above, and/or (2R,cis)-(2-aryl-2-halomethyl-1,3-dioxolan-4-yl)methyl sulfonate represented by the formula [XIX], and
a step of isolating (2R,cis)-(2-aryl-2-halomethyl-1,3-dioxolan-4-yl)methyl sulfonate represented by the formula [XIX] by recrystallization from a mixture containing (2S,trans)-(2-aryl-2-halomethyl-1,3-dioxolan-4-yl)methyl sulfonate represented by the formula [XVIII] and (2R,cis)-(2-aryl-2-halomethyl-1,3-dioxolan-4-yl)methyl sulfonate represented by the formula [XIX].

**24.** A method of producing (2R,cis)-(2-aryl-2-halomethyl-1,3-dioxolan-4-yl)methyl carboxylate represented by the formula [XV], comprising isolating (2R,cis)-(2-aryl-2-halomethyl-1,3-dioxolan-4-yl)methyl carboxylate represented by the formula [XV] by crystallization from a mixture containing (2S,trans)-(2-aryl-2-halomethyl-1,3-dioxolan-4-yl)methyl carboxylate represented by the formula [XIV] and (2R,cis)-(2-aryl-2-halomethyl-1,3-dioxolan-4-yl)methyl carboxylate represented by the formula [XV], which are obtained by the production method according to claim 22.

**25.** A method of producing (2R,cis)-(2-aryl-2-halomethyl-1,3-dioxolan-4-yl)methyl carboxylate represented by the formula [XV], comprising isomerizing (2S,trans)-(2-aryl-2-halomethyl-1,3-dioxolan-4-yl)methyl carboxylate represented by the formula [XIV], which is contained in the mother liquid of the recrystallization in the method according to claim 24, in the presence of an acid catalyst.

**26.** The production method according to claim 25, which is performed in at least one kind of solvent selected from aliphatic hydrocarbon solvents, aromatic hydrocarbon solvents, halogenated hydrocarbon solvents, nitrile solvents, ester solvents and acid catalysts.

**27.** The production method according to claim 25 or 26, comprising a step of isolating (2R,cis)-(2-aryl-2-halomethyl-1,3-dioxolan-4-yl)methyl carboxylate represented by the formula [XV] after the isomerization step.

**28.** The production method according to claim 27, comprising isolation by recrystallization.

**29.** A method of producing (2R,cis)-(2-aryl-2-halomethyl-1,3-dioxolan-4-yl)methyl carboxylate represented by the formula [XV], comprising reacting a mixture containing (2S,trans)-(2-aryl-2-halomethyl-1,3-dioxolan-4-yl)methyl carboxylate represented by the formula [XIV] and (2R,cis)-(2-aryl-2-halomethyl-1,3-dioxolan-4-yl)methyl carboxylate represented by the formula [XV], which are obtained by the production method according to claim 22, in the presence of an acid catalyst to isomerize (2S,trans)-(2-aryl-2-halomethyl-1,3-dioxolan-4-yl)methyl carboxylate represented by the formula [XIV] to (2R,cis)-(2-aryl-2-halomethyl-1,3-dioxolan-4-yl)methyl carboxylate represented by the formula [XV], while simultaneously precipitating (2R,cis)-(2-aryl-2-halomethyl-1,3-dioxolan-4-yl)methyl carboxylate represented by the formula [XV] as crystals.

**30.** The production method according to claim 29, which is performed in at least one kind of solvent selected from aliphatic hydrocarbon solvents, aromatic hydrocarbon solvents, halogenated hydrocarbon solvents, nitrile solvents, ester solvents and acid catalysts.

**31.** The production method according to any of claims 25 to 30, wherein the acid catalyst is a Lewis acid or an organic acid.

**32.** A method of producing (2R,cis)-(2-aryl-2-halomethyl-1,3-dioxolan-4-yl)methyl sulfonate represented by the formula [XIX]

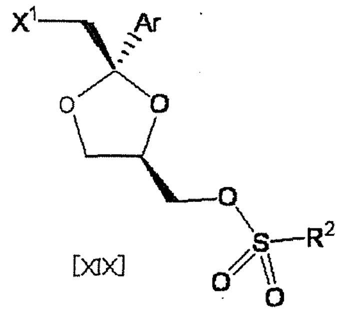
[XIX]

wherein Ar is a substituted or unsubstituted aromatic ring group, or a substituted or unsubstituted heteroaromatic ring group, and $X^1$ is a halogen atom, comprising a step of hydrolyzing (2R,cis)-(2-aryl-2-halomethyl-1,3-dioxolan-4-yl)methyl carboxylate represented by the formula [XV], which is obtained by the production method according to any of claims 24 to 31, to give (2R,cis)-2-aryl-2-halomethyl-1,3-dioxolan-4-methanol represented by the formula [XVII]

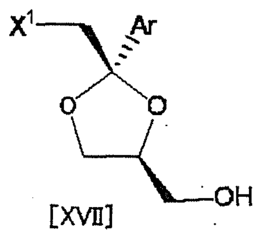
[XVII]

wherein each symbol is as defined above, and
a step of converting (2R,cis)-2-aryl-2-halomethyl-1,3-dioxolan-4-methanol represented by the formula [XVII] to (2R, cis)-(2-aryl-2-halomethyl-1,3-dioxolan-4-yl)methyl sulfonate represented by the formula [XIX].

**33.** The production method according to any of claims 1 to 32, wherein aryl(halomethyl)ketone represented by the formula [I] is (4-chlorophenyl)-2-bromomethylketone, (2,4-dichlorophenyl)-2-bromomethylketone, (4-fluorophenyl)-2-bromomethylketone, (2,4-difluorophenyl)-2-bromomethylketone, (4-chloro-2-trifluoromethylphenyl)-2-bromomethylketone, phenyl-2-bromomethylketone, (4-methylphenyl)-2-bromomethylketone, (4-biphenyl)-2-bromomethylketone, (2-naphthyl)-2-bromomethylketone, (4-methoxyphenyl)-2-bromomethylketone, (4-phenoxyphenyl)-2-bromomethylketone, (4-nitrophenyl)-2-bromomethylketone, (2-thienyl)-2-bromomethylketone, (4-chlorophenyl)-2-chloromethylketone, phenyl-2-chloromethylketone or (4-methylphenyl)-2-chloromethylketone.

**34.** A method of producing (2S,cis)-(2-aryl-2-halomethyl-1,3-dioxolan-4-yl)methyl carboxylate represented by the for-

mula [VI]

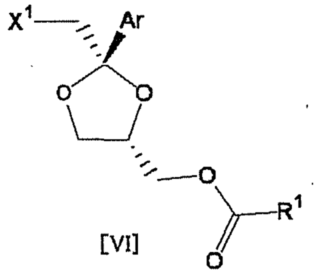

[VI]

wherein Ar is a substituted or unsubstituted aromatic ring group, or a substituted or unsubstituted heteroaromatic ring group, $X^1$ is a halogen atom, and $R^1$ is a substituted or unsubstituted hydrocarbon group, or a substituted or unsubstituted aromatic ring group, comprising a step of isomerizing (2R,trans)-(2-aryl-2-halomethyl-1,3-dioxolan-4-yl)methyl carboxylate represented by the formula [V]

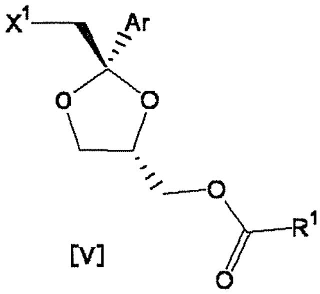

[V]

wherein each symbol is as defined above, to (2S,cis)-(2-aryl-2-halomethyl-1,3-dioxolan-4-yl)methyl carboxylate represented by the formula [VI], in the presence of an acid catalyst.

**35.** A method of producing (2R,cis)-(2-aryl-2-halomethyl-1,3-dioxolan-4-yl)methyl carboxylate represented by the formula [XV]

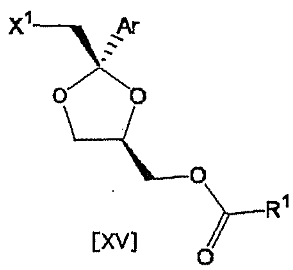

[XV]

wherein Ar is a substituted or unsubstituted aromatic ring group, or a substituted or unsubstituted heteroaromatic ring group, $X^1$ is a halogen atom, and $R^1$ is a substituted or unsubstituted hydrocarbon group, or a substituted or unsubstituted aromatic ring group, comprising a step of isomerizing (2S,trans)-(2-aryl-2-halomethyl-1,3-dioxolan-4-yl)methyl carboxylate represented by the formula [XIV]

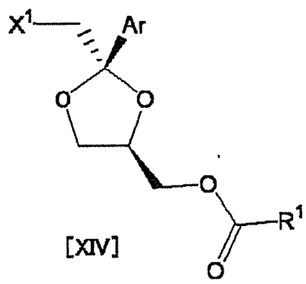

[XIV]

wherein each symbol is as defined above, to (2R,cis)-(2-aryl-2-halomethyl-1,3-dioxolan-4-yl)methyl carboxylate represented by the formula [XV], in the presence of an acid catalyst.

**36.** The production method according to claim 34 or 35, wherein, in the optically active (2-aryl-2-halomethyl-1,3-dioxolan-4-yl)methyl carboxylates represented by the formulas [V], [VI], [XIV] and [XV], Ar is an aromatic ring group or a halogen-substituted aromatic ring group, and $X^1$ is a chlorine atom or a bromine atom.

**37.** The production method according to any of claims 34 to 36, which is performed in at least one kind of solvent selected from aliphatic hydrocarbon solvents, aromatic hydrocarbon solvents, halogenated hydrocarbon solvents, nitrile solvents, ester solvents and acid catalysts.

**38.** The production method according to any of claims 34 to 37, wherein the acid catalyst is a Lewis acid or an organic acid.

**39.** The production method according to any of claims 34 to 38, comprising a step of isolating cis-(2-aryl-2-halomethyl-1,3-dioxolan-4-yl)methyl carboxylate after the isomerization step.

**40.** The production method according to any of claims 34 to 39, comprising isolation by recrystallization.

**41.** The production method according to any of claims 34 to 40, wherein, in the isomerization step, isomerization is performed while precipitating cis-(2-aryl-2-halomethyl-1,3-dioxolan-4-yl)methyl carboxylate as crystals.

**42.** The production method according to any of claims 34 to 41, comprising a step of quenching the acid catalyst with a base after completion of the reaction.

**43.** A (2S,cis)-(2-aryl-2-halomethyl-1,3-dioxolan-4-yl)methyl carboxylate represented by the formula [VI]

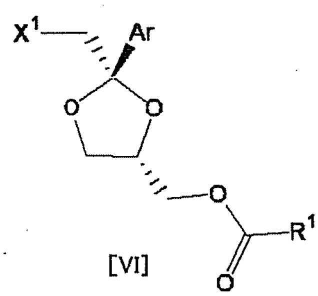

[VI]

wherein Ar is a substituted or unsubstituted aromatic ring group, or a substituted or unsubstituted heteroaromatic ring group, $X^1$ is a halogen atom, and $R^1$ is a substituted or unsubstituted hydrocarbon group, or a substituted or unsubstituted aromatic ring group, having a content of a halogen-exchanged compound wherein $X^1$ is exchanged with other halogen group of not more than 0.2%.

**44.** A (2R,cis)-(2-aryl-2-halomethyl-1,3-dioxolan-4-yl)methyl carboxylate represented by the formula [XV]

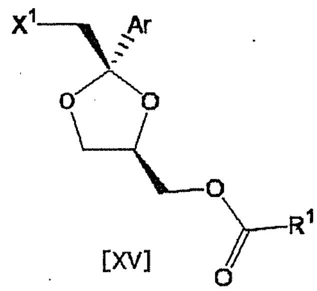

[XV]

wherein Ar is a substituted or unsubstituted aromatic ring group, or a substituted or unsubstituted heteroaromatic ring group, $X^1$ is a halogen atom, and $R^1$ is a substituted or unsubstituted hydrocarbon group, or a substituted or unsubstituted aromatic ring group, having a content of a halogen-exchanged compound wherein $X^1$ is exchanged with other halogen group of not more than 0.2%.

**45.** The compound according to claim 43 or 44, wherein $X^1$ is a halogen atom other than a chlorine atom, and the content of the halogen-exchanged compound, wherein $X^1$ is a chlorine atom, is not more than 0.2%.

**46.** The compound according to claim 43 or 44, wherein $X^1$ is a bromine atom, Ar is 4-chlorophenyl, 2,4-dichlorophenyl, 4-fluorophenyl, 2,4-difluorophenyl, 4-chloro-2-trifluoromethylphenyl, phenyl, 4-methylphenyl, 4-biphenyl, 2-naphthyl, 4-methoxyphenyl, 4-phenoxyphenyl, 4-nitrophenyl or 2-thienyl, $R^1$ is phenyl, methyl, ethyl, 4-nitrophenyl, 4-methoxyphenyl, 4-chlorophenyl or heptyl, and the content of the halogen-exchanged compound, wherein $X^1$ is a chlorine atom, is not more than 0.2%.

**INTERNATIONAL SEARCH REPORT**

International application No.
PCT/JP2009/055714

A. CLASSIFICATION OF SUBJECT MATTER
*C07D317/16*(2006.01)i, *C07D317/18*(2006.01)i, *C07D409/04*(2006.01)i

According to International Patent Classification (IPC) or to both national classification and IPC

B. FIELDS SEARCHED

Minimum documentation searched (classification system followed by classification symbols)
C07D317/16, C07D317/18, C07D409/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CAplus(STN), CASREACT(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | CISZEWNSKI, L., Effect of reaction conditions on the nucleophilic substitution of *R*, *S*, *cis-trans*-trisubstituted 1, 3-dioxolane, Polish Journal of Chemistry, 62(1/3), pp.179-186 (1988) | 1-42 |
| A | WOLINSKI, J., *et al.*, Search for anticholinergic agents, Acta Polon. Pharm., 33(6), pp.695-700 (1976) | 1-42 |
| X<br>A | JP 2002-535328 A (Korea Research Institute), 22 October, 2002 (22.10.02),<br>& WO 2000/043390 A1 & AU 200030809 A<br>& KR 2000051143 A & EP 1144406 A1<br>& KR 2001101593 A & US 6376497 B1<br>& JP 3386795 B2 & EP 1144406 B1<br>& DE 60004165 E & KR 462072 B | 43-46<br>1-42 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 May, 2009 (15.05.09) | 26 May, 2009 (26.05.09) |
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2009/055714

C (Continuation). DOCUMENTS CONSIDERED TO BE RELEVANT

| Category* | Citation of document, with indication, where appropriate, of the relevant passages | Relevant to claim No. |
|---|---|---|
| X<br>A | WO 96/029325 A1 (LAB MENARINI S.A.), 26 September, 1996 (26.09.96), & AU 9650039 A & ES 2112151 A1 & ES 2112151 B1 | 43-46<br>1-42 |
| X<br>A | Isao TAKEUCHI et al., "*cis*-1-[2-Phenyl-4-(phenoxy or Phenylthio)methyl-1, 3- dioxolan-2-ylmethyl]-1*H*-imidazole Yudotai no Gosei to Sono Kokin Sayo ni Tsuite", Journal of the Pharmaceutical Society of Jap, 105(6), pages 554 to 561 (1985) | 43-46<br>1-42 |
| X<br>A | ROTSTEIN, D.M., *et al.*, Stereoisomers of Ketoconazole: Preparation and Biological Activity, J. Med. Chem., 35(15), pp.2818-2825 (1992) | 43-46<br>1-42 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008200225 A
- JP 2008235746 A


### Non-patent literature cited in the description


- **Rotstein, D.M. et al.** *Journal of Medicinal Chemistry,* 1992, vol. 35, 2818-2825
- **Camps, P. et al.** *Tetrahedron Asymmetry,* 1995, vol. 6, 1283-1294
- **Jacobsen, E.N.** *Science,* 1997, vol. 277, 936-938
- **Bauer, L. et al.** *J. Heterocycl. Chem.,* 1990, vol. 27, 2053-2061